# EUROPÄISCHE PATENTSCHRIFT

(11) **EP 2 231 654 B1**
(45) Veröffentlichungstag und Bekanntmachung des Hinweises auf die Patenterteilung: **03.07.2013**
(21) Anmeldenummer: 08859757.0
(22) Anmeldetag: 28.11.2008
(51) Int. Cl.: C07D 417/14, C07D 417/04, A61K 31/427, A61P 35/00, A61P 37/08

(54) **NEUE 2-SUBSTITUIERTE TIAZOL-4-CARBONSÄUREAMID-DERIVATE, DEREN HERSTELLUNG UND VERWENDUNG ALS ARZNEIMITTEL**
NEW 2-SUBSTITUTED TIAZOL-4-CARBOXYLIC ACID DERIVATIVES, THEIR MANUFACTURE AND USE AS MEDICINE
NOUVEAUX DÉRIVÉS DE 2-THIAZOL-4-AMIDES D'ACIDE CARBOXYLIQUE SUBSTITUÉS, LEUR FABRICATION ET LEUR UTILISATION EN TANT QUE MÉDICAMENT

(30) Priorität: 10.12.2007 EP 07076070
(43) Veröffentlichungstag der Anmeldung: 29.09.2010
(73) Patentinhaber: Bayer Intellectual Property GmbH, 40789 Monheim (DE)
(72) Erfinder: BOTHE, Ulrich, 13187 Berlin (DE); VON BONIN, Arne, 16548 Glienicke-Nordbahn (DE); NGUYEN, Duy, 10179 Berlin (DE); BÖMER, Ulf, 16548 Glienicke (DE); GUENTHER, Judith, 13187 Berlin (DE)
(74) Vertreter: BIP Patents
(86) Internationale Anmeldenummer: PCT/EP2008/010105
(87) Internationale Veröffentlichungsnummer: WO 2009/074234

(56) Entgegenhaltungen:
- EP-A1- 1 832 586
- DATABASE CHEMCATS [Online] CHEMICAL ABSTRACTS SERVICE,COLUMBUS,OHIO,US; XP002487587 gefunden im STN & "aurora screening library" 6. September 2007 (2007-09-06), AURORA FINE CHEMICALS , AUSTRIA

## Beschreibung

Die vorliegende Erfindung betrifft 2-substituierte Tiazol-4-carbonsäureamid -Derivate, die Verwendung als Medikament zur Behandlung verschiedener Erkrankungen finden.

### Biologischer Hintergrund

Für zahlreiche chronisch entzündliche Erkrankungen, wie z.B. Rheumatoider Arthritis, Morbus Crohn, Asthma und Multipler Sklerose, ist ein überreagierendes Immunsystem mit verantwortlich. Durch ein vermehrtes Freisetzen von proinflammatorischen Zytokinen kommt es zu Schädigungen von körpereigenen Gewebestrukturen. Hierbei ist das Zusammenspiel von angeborenem und adaptiven Immunsystem von zentraler Bedeutung (Akira et al., 2001). Eine Modulation des Immunsystems mit Substanzen, die mit der Aktivierung von Zellen des angeborenen und/oder des adaptiven Immunsystem interferieren, wirkt anti-inflammatorisch und kann damit den pathologischert Phänotyp in den oben exemplarisch genannten Erkrankungen abmildern.
Die angeborene Immunität ("innate immunity") beruht darauf, daß Mikroorganismen wie Bakterien und Viren bestimmte inherente Merkmale aufweisen, durch die sie vom Immunsystem erkannt werden und dieses anschließend aktivieren. Erkannt werden bestimmte Pathogen assozierte Muster ("pathogen associated molecular pattern, PAMPs"). PAMPs werden von den "Pattern recognition receptors" (PRR) erkannt, zu denen auch Toll Like Rezeptoren (TLR) gehören (Janeway und Medzhitov, 2002). TLRs sind Homologe zum Drosophila-Rezeptorprotein "toll". Der Mensch hat zehn verschiedene TLRs. TLR eins und sechs sind Corezeptoren für TLR2. TLR2 erkennt u.a. Lipoproteine und Lipopeptide. TLR3 erkennt doppelsträngige RNA. TLR4 erkennt u.a. LPS von gram-negativen und Lipoteichonsäure von gram-positiven Bakterien. TLR5 erkennt Flagellin. TLR9 erkennt CpG-Motive in bakterieller DNA (O'Neill, 2006). Corezeptoren können die Erkennungsfähigkeiten von TLRs weiter verändern (Jiang et al., 2005).

### IL-1/-18, TLR Signaltransduktion

TLRs sind in der Signalübertragung mit IL-1 / IL-18 Zytokinrezeptoren verwandt. IL-1 ("endogenes Pyrogen") stimuliert stark Entzündung und induziert Fieber.
Mitglieder der IL-1 R/TLR Superfamilie haben eine TIR Domäne (Toll/IL1 Receptor). Die TIR Domäne ist etwa 200 Aminosäuren lang und enthält drei konservierte Sequenzmotive. TIR-Domänen tragende Proteine binden über eine Protein-Protein Interaktion (O'Neill et al., 2005). Die Subklasse eins (IL-1R Familie) enthält drei Igartige-Domänen, der Rezeptor ist ein Heterodimer. Dazu gehören die IL-1 Rezeptoren eins und zwei, der Co-Rezeptor IL-1 RAcP und die entsprechenden Proteine des IL-18 Systems. Die Subklasse zwei (TLR-Familie) enthält Leucin-rich Motife. Toll-like Rezeptoren bilden Homo- oder Heterodimere.

Nach Aktivierung der TLR bzw. IL-1, -18 Rezeptoren durch die entsprechenden Liganden wird eine mehrstufige Signalkaskade in Gang gesetzt. Der TLR bzw. IL-1/- 18 Rezeptorkomplex wechselwirkt über TIR/TIR Kontakte mit dem Adaptorprotein MyD88. Die "IL-1 associated receptor kinase" (IRAK-1) hat normalerweise Tollip (Toll interacting protein) gebunden, das wahrscheinlich als ein abschwächendes Molekül ("silencer") wirkt. IRAK/Tollip bindet an den aktiven TLR/IL-1 R Komplex. MyD88 verdrängt Tollip wodurch IRAK1 und IRAK-4 aktiviert wird, höchstwahrscheinlich als Dimer durch Transphosphorylierung. Aktives IRAK verlässt den Rezeptor und bindet im Cytoplasma an das Adaptermolekül TRAF (Barton und Medzhitov, 2003). Über TRAF werden weitere Proteine ubiquitiniliert. Über einen unbekannten Mechanismus führt Ub-TRAF zur Autophosphorylierung der S/T-Kinase TAK1 (eine MAP-Kinase Kinasekinase). TAK1 phosphoryliert IκB (NF-κB Aktivierung) und MKK6. Letztere ist für die Aktivierung der MAP-Kinasen p38 und JNK verantwortlich. NF-κB wurde als Nuclear Factor für die Expression der leichten Antikörperkette Kappa in B-Zellen identifiziert, ist aber an der Regulation vieler anderer Gene ebenfalls beteiligt. NF-κB wird im inaktiven Zustand im Cytoplasma zurückgehalten, wo es an den Inhibitor IκB gebunden ist (Deng et al., 2000). Phosphorylierung von IκB bewirkt, daß der Inhibitor IkB proteolytisch abgebaut wird und der Transkriptionsfaktor in den Kern wandern kann. NF-κB ist ein Heterodimer aus den Untereinheiten p65 (Rel) und p50 (Bäuerle und Henkel, 1994). Es gibt mehrere Mitglieder dieser Familie, die auf unterschiedliche Weise zusammenwirken können. NF-κB alleine kann Transkription nicht auslösen. Für Genaktivierung sind transkriptionelle Coaktivatoren erforderlich, wie etwa p300 oder CBT (Akira und Takeda, 2004).
Nach erfolgter Aktivierung von TIR-Domäne enthaltenden Rezeptoren kommt es unter anderem zur Freisetzung von inflammatorischen Zytokinen, wie z.B. IL-1, IL-6, IL-23 und TNF-alpha (Adachi et al., 1998).

Den strukturell naheliegenden Stand der Technik bilden die Strukturen folgender Patentanmeldungen:

In WO2007/016292 werden N-Aryl-2-arylthiazol-4-carbonsäureamid-Derivate als Biofilmmodulatoren (Modulatoren von Bakterienfilmen) beschrieben, die aufgrund des bicyclischen C-D-Ringsystemes unterschiedlich von den hier beanspruchten Verbindungen sind.
In WO2007/035478 werden Verbindungen offenbart, die anstelle einer Carboxamidgruppe eine Carboxylgruppe in ortho-Positon aufweisen.
In US 6,274,738 werden N-Aryl-2-pyridylthiazol-4-carbonsäureamid-Derivate als Verbindungen beschrieben, die die DNA Primase modulieren. Allerdings weisen diese Verbindungen an der N-Arylgruppe keine Aminocarbonylgruppe auf, die in ortho-Postion bezüglich der Pyridylthiazol-4-carbonsäureamideinheit steht.
Darüber hinaus werden in US 4,879295 N-Tetrazolylthiazolcarboxamide offenbart. Thiazolamidderivate werden in WO2006/122011 als Inhibitoren der Virusreplikation genannt. In WO2005/048953 werden Thiazolamidderivate verknüpft mit einer Isoxazoleinheit als Kinaseinhibitoren berschrieben. Die Strukturen unterscheiden sich allerdings von den Strukturen der vorliegenden Erfindung.
Auch die in der WO2007/052882 offenbarten Verbindungen weisen keine Aminocarbonylgruppe in ortho-Position zur Aminocarbonylthiazolgruppe auf. Besonderes Merkmal der in WO2004072025 beschriebenen Verbindungen ist im Gegensatz zu den hier offenbarten Strukturen ein Pyrrolidinring, der zusätzlich noch mit einem weiteren Substituenten (wie z. B. einer Dimethylaminogruppe) über ein Stickstoffatom verknüpft vorliegt.

In WO200512256, WO200677424, WO2006077425 und WO200677428 werden Pyrazolderivate als Kinaseinhibitoren offenbart. Unter anderem sind auch Strukturen beschrieben, bei denen ein Thiazolamid mit der Pyrazoleinheit verknüpft ist, wobei allerdings keines der Pyrazol-Stickstoffatome methyliert vorliegt und die Pyrazoleinheit auch nicht mit einer Aminocarbonylgruppe (C(O)NH₂) substituiert ist.

Pyrazolamide, die aber gleichzeitig mit einer Harnstoffeinheit verknüpft vorliegen, werden in WO2005/37797 beschrieben.

In WO2005/115986 werden Pyridinamide beansprucht, wobei aber keine Verknüpfungen mit schwefelhaltigen Heterocyclen wie Thiazol vorgesehen sind.

Strukturell unterschiedlich zu den hier beschriebenen Verbindungen sind auch die in WO2005/049604 beschriebenen Pyridinamide, aufgrund der Verknüpfung mit einem sauerstoffsubstituierten Phenylring. In EP 1832586 werden Thiazolpiperidine derivate zur Behandlung van Leberkrebs offenbart.

In EP1666455 werden Amide mit zusätzlicher Carboxamidstruktur beschrieben, wobei der Substituent R1 keine Aminocarbonylgruppe sein kann.

Ausgehend von diesem Stand der Technik besteht die Aufgabe der vorliegenden Erfindung, weitere Strukturen für die Therapie bereitzustellen, insbesondere für die Immunmodulation.

Die Aufgabe wird gelöst durch Verbindungen der allgemeinen Formel (I),
mit den Bausteinen A, B, C und D , 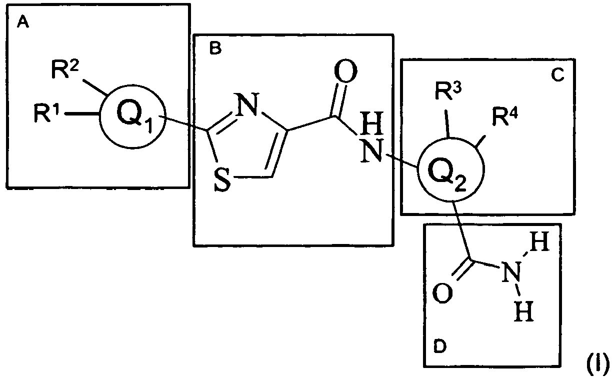 in der
die Bausteine B und D in ortho-Stellung zueinander stehen und
- Q₁: für einen Heteroarylring mit 5 Ringatomen steht,
- R¹ und R²: unabhängig voneinander stehen für
(i) Wasserstoff, Hydroxy, Nitro, Halogen, Cyano, -CF₃, -NR⁵R⁶ oder
(ii) -C(O)-OR¹⁰, -C(O)-R⁷, -C(O)-C₁-C₃-Fluoralkyl, -C(O)-NR⁵R⁶, -NH-C(O)-R⁷ oder
(iii) einen C₁-C₆-Alkyl-, C₂-C₆ -Alkenyl-, C₂-C₆-Alkinyl-, C₁-C₆-Alkoxy-, C₁-C₆-Fluoralkyl- oder einen C₁-C₆-Fluoralkoxyrest,
jeweils gegebenenfalls mit -C₁-C₃-Alkoxy, Hydroxy, -C(O)-OR¹⁰ oder -NR⁸R⁹ ein- oder mehrfach, gleich oder verschieden substituiert, oder
(iv) -O-SO₂-NR⁵R⁶, -SO₂-R⁷, -SO₂-NR⁵R⁶,
- Q₂: für einen Heteroaryl- oder einen hydrierten bizyklischen Heteroarylring steht;
- R³ und R⁴: unabhängig voneinander stehen für
(i) Wasserstoff, Halogen oder -NR¹¹R¹², oder
(ii) einen C₁-C₆-Alkyl- oder C₁-C₆-Alkoxyrest, die jeweils gegebenenfalls ein- oder mehrfach, gleich oder verschieden mit Hydroxy, C₁-C₃-Alkoxy, -NR⁸R⁹ oder Heterocyclyl substituiert sind, wobei der Heterocylylring ein- oder mehrfach, gleich oder verschieden substituiert sein kann mit C₁-C₃-Alkyl oder -C(O)-R⁷ , wobei
- R⁵ und R⁶: unabhängig voneinander stehen für Wasserstoff oder einen C₁-C₆ Alkylrest, der gegebenenfalls ein- oder mehrfach, gleich oder verschieden mit Hydroxy, -C₁-C₃-Alkoxy, -NR⁸R⁹ oder Heterocyclyl substituiert ist, wobei der Heterocylylring ein- oder mehrfach, gleich oder verschieden substituiert sein kann mit C₁-C₃-Alkyl oder -C(O)-R⁷, oder
- R⁵ und R⁶: bilden alternativ gemeinsam mit dem Stickstoffatom einen 5- bis 7-gliedrigen Ring, der gegebenenfalls zusätzlich zum Stickstoffatom ein weiteres Heteroatom enthält und der gegebenenfalls mit C₁-C₆-Alkyl und/oder mit -C(O)-R⁷ ein- oder mehrfach, gleich oder verschieden substituiert ist, und
- R⁷: für einen C₁-C₆ Alkylrest steht, und
- R⁸, R⁹, R¹⁰: unabhängig voneinander stehen für Wasserstoff oder einen C₁-C₆ Alkylrest, und
- R¹¹ und R¹²: unabhängig voneinander stehen für Wasserstoff oder einen C₁-C₆ Alkylrest, der gegebenenfalls ein- oder mehrfach, gleich oder verschieden mit Hydroxy, -C₁-C₃-Alkoxy, -NR⁸R⁹ oder Heterocyclyl substituiert ist, wobei der Heterocylylring ein- oder mehrfach, gleich oder verschieden substituiert sein kann mit C₁-C₃-Alkyl oder -C(O)-R⁷, oder
sowie deren Salze, Enantiomere und Diastereomere.

Der Erfindung liegen folgende Definitionen zu Grunde:

### Cₙ-Alkyl:

Monovalenter, geradkettiger oder verzweigter, gesättigter Kohlenwasserstoffrest mit n Kohlenstoffatomen.

Ein C₁-C₆ Alkylrest umfasst unter anderem beispielsweise:
Methyl-, Ethyl-, Propyl-, Butyl-, Pentyl-,Hexyl-, iso-Propyl-, *iso*-Butyl-, *sec*-Butyl, *tert-*Butyl-, *iso*-Pentyl-, 2-Methylbutyl-, 1-Methylbutyl-, 1-Ethylpropyl-, 1,2-Dimethylpropyl, *neo*-Pentyl-, 1,1-Dimethylpropyl-, 4-Methylpentyl-, 3-Methylpentyl-, 2-Methylpentyl-, 1-Methylpentyl-, 2-Ethylbutyl-, 1-Ethylbutyl-, 3,3-Dimethylbutyl-, 2,2-Dimethylbutyl-, 1,1-Dimethylbutyl-, 2,3-Dimethylbutyl-, 1,3-Dimethylbutyl- 1,2-Dimethylbutyl-.
Bevorzugt ist ein Methyl-, Ethyl-, Propyl- oder Isopropylrest.

### Cₙ-Fluoralkyl:

Monovalenter, geradkettiger oder verzweigter, gesättigter, vollständig oder teilweise fluorierter Kohlenwasserstoffrest mit n Kohlenstoffatomen.

Ein C₁-C₆ Fluoralkylrest umfasst unter anderem beispielsweise:
Trifluormethyl, Difluormethyl, Monofluormethyl, Pentafluorethyl, Heptafluorpropyl, 2,2,2-Trifluorethyl, 3,3,3-Trifluorpropyl, 5,5,6,6,6-Pentafluorhexyl, Pentafluorallyl, 1,1,1,3,3,3-Hexafluor-2-propyl.
Bevorzugt ist ein Trifluormethyl, 2,2,2-Trifluorethyl und Pentafluorethylrest.

### Cₙ-Alkenyl:

monovalenter, geradkettiger oder verzweigter Kohlenwasserstoffrest mit n Kohlenstoffatomen und mindestens einer Doppelbindung.

Ein C₂-C₆ Alkenylrest umfasst unter anderem beispielsweise:
Vinyl-, Allyl-, (E)-2-Methylvinyl-, (Z)-2-Methylvinyl-, Homoallyl-, (E)-But-2-enyl-, (Z)-But-2-enyl-, (E)-But-1-eny)-, (Z)-But-1-eny)-, Pent-4-enyl-, (E)-Pent-3-enyl-, (Z)-Pent-3-enyl-, (E)-Pent-2-enyl-, (Z)-Pent-2-enyl-, (E)-Pent-1-enyl-, (Z)-Pent-1-enyl-, Hex-5-enyl-, (E)-Hex-4-enyl-, (Z)-Hex-4-enyl-, (E)-Hex-3-enyl-, (Z)-Hex-3-enyl-, (E)-Hex-2-enyl-, (Z)-Hex-2-enyl-, (E)-Hex-1-enyl-, (Z)-Hex-1-enyl-, Isopropenyl-, 2-Methylprop-2-enyl-, 1-Methylprop-2-enyl-, 2-Methylprop-1-enyl-, (E)-1-Methylprop-1-enyl-, (Z)-1-Methylprop-1-enyl-, 3-Methylbut-3-enyl-, 2-Methylbut-3-enyl-, 1-Methylbut-3-enyl-, 3-Methylbut-2-enyl-, (E)-2-Methylbut-2-enyl-, (Z)-2-Methylbut-2-enyl-, (E)-1-Methylbut-2-enyl-, (Z)-1-Methylbut-2-enyl-, (E)-3-Methylbut-1-enyl-, (Z)-3-Methylbut-1-enyl-, (E)-2-Methylbut-1-enyl-,
(Z)-2-Methylbut-1-enyl-, (E)-1-Methylbut-1-enyl-, (Z)-1-Methylbut-1-enyl-, 1,1-Dimethylprop-2-enyl-, 1-Ethylprop-1-enyl-, 1-Propylvinyl-, 1-Isopropylvinyl-, 4-Methylpent-4-enyl-, 3-Methylpent-4-enyl-, 2-Methylpent-4-enyl-, 1-Methylpent-4-enyl-, 4-Methylpent-3-enyl-, (E)-3-Methylpent-3-enyl-, (Z)-3-Methylpent-3-enyl-, (E)-2-Methylpent-3-enyl-, (Z)-2-Methylpent-3-enyl-, (E)-1-Methylpent-3-enyl-, (Z)-1-Methylpent-3-enyl-, (E)-4-Methylpent-2-enyl-, (Z)-4-Methylpent-2-enyl-, (E)-3-Methylpent-2-enyl-, (Z)-3-Methylpent-2-enyl-, (E)-2-Methylpent-2-enyl-, (Z)-2-Methylpent-2-enyl-, (E)-1-Methylpent-2-enyl-, (Z)-1-Methylpent-2-enyl-, (E)-4-Methylpent-1-enyl-, (Z)-4-Methylpent-1-enyl-, (E)-3-Methylpent-1-enyl-, (Z)-3-Methylpent-1-enyl-, (E)-2-Methylpent-1-enyl-, (Z)-2-Methylpent-1-enyl-, (E)-1-Methylpent-1-enyl-, (Z)-1-Methylpent-1-enyl-, 3-Ethylbut-3-enyl-, 2-Ethylbut-3-enyl-, 1-Ethylbut-3-enyl-, (E)-3-Ethylbut-2-enyl-, (Z)-3-Ethylbut-2-enyl-, (E)-2-Ethylbut-2-enyl-, (Z)-2-Ethylbut-2-enyl-, (E)-1-Ethylbut-2-enyl-, (Z)-1-Ethylbut-2-enyl-, (E)-3-Ethylbut-1-enyl-, (Z)-3-Ethylbut-1-enyl-, 2-Ethylbut-1-enyl-, (E)-1-Ethylbut-1-enyl-, (Z)-1-Ethylbut-1-enyl-, 2-Propylprop-2-enyl-, 1-Propylprop-2-enyl-, 2-lsopropylprop-2-enyl-, 1-Isopropylprop-2-enyl-, (E)-2-Propylprop-1-enyl-, (Z)-2-Propylprop-1-enyl-, (E)-1-Propylprop-1-enyl-, (Z)-1-Propylprop-1-enyl-, (E)-2-Isopropylprop-1-enyl-, (Z)-2-Isopropylprop-1-enyl-, (E)-1-Isopropylprop-1-enyl-, (Z)-1-Isopropylprop-1-enyl-, (E)-3,3-Dimethylprop-1-enyl-, (Z)-3,3-Dimethylprop-1-enyl-, 1-(1,1-Dimethylethyl)ethenyl.

Bevorzugt ist ein Vinyl- oder Allylrest.

### Cₙ-Alkinyl:

Monovalenter, geradkettiger oder verzweigter Kohlenwasserstoffrest mit n Kohlenstoffatomen und mindestens einer Dreifachbindung.

Ein C₂-C₆ Alkinylrest umfasst unter anderem beispielsweise:
Ethinyl-, Prop-1-inyl-, Prop-2-inyl-, But-1-inyl-, But-2-inyl-, But-3-inyl-, Pent-1-inyl-, Pent-2-inyl-, Pent-3-inyl-, Pent-4-inyl-, Hex-1-inyl-, Hex-2-inyl-, Hex-3-inyl-, Hex-4-inyl-, Hex-5-inyl-, 1-Methylprop-2-inyl-, 2-Methylbut-3-inyl-, 1-Methylbut-3-inyl-, 1-Methylbut-2-inyl-, 3-Methylbut-1-inyl-, 1-Ethylprop-2-inyl-, 3-Methylpent-4-inyl, 2-Methylpent-4-inyl, 1-Methylpent-4-inyl, 2-Methylpent-3-inyl, 1-Methylpent-3-inyl, 4-Methylpent-2-inyl, 1-Methylpent-2-inyl, 4-Methylpent-1-inyl, 3-Methylpent-1-inyl, 2-Ethylbut-3-inyl-, 1-Ethylbut-3-inyl-, 1-Ethylbut-2-inyl-, 1-Propylprop-2-inyl-, 1-Isopropylprop-2-inyl-, 2,2-Dimethylbut-3-inyl-, 1,1-Dimethylbut-3-inyl-, 1,1-Dimethylbut-2-inyl- oder eine 3,3-Dimethylbut-1-inyl-.

Bevorzugt ist ein Ethinyl-, Prop-1-inyl- oder Prop-2-inylrest.

### Cₙ-Cycloalkyl:

Monovalenter, cyclischer Kohlenwasserstoffring mit n Kohlenstoffatomen.

C₃-C₇-Cyclolalkylring umfasst:
Cyclopropyl, Cyclobutyl, Cyclopentyl, Cyclohexyl- und Cycloheptyl.

Bevorzugt ist ein Cyclopropyl-, Cyclobutyl-, Cylopentyl- oder ein Cyclohexylring.

### Cₙ-Alkoxy:

Geradkettiger oder verzweigter Cₙ-Alkyletherrest der Formel -OR mit R=Alkyl.

### Cₙ-Fluoralkoxy:

Geradkettiger oder verzweigter Cₙ-Fluoralkyletherrest der Formel -OR mit R=Cₙ-Fluoralkyl.

### Cₙ-Aryl

Cₙ-Aryl ist ein monovalentes, aromatisches Ringsystem ohne Heteroatom mit n Kohlenwasserstoffatomen.
C₆-Aryl ist gleich Phenyl. C₁₀-Aryl ist gleich Naphthyl.
Bevorzugt ist Phenyl.

### Heteroatome

Unter Heteroatomen sind Sauerstoff-, Stickstoff- oder Schwefel-Atome zu verstehen.

### Heteroaryl

Heteroaryl ist ein monovalentes, aromatisches Ringsystem mit mindestens einem von einem Kohlenstoff verschiedenen Heteroatom. Als Heteroatome können Stickstoffatome, Sauerstoffatome und/oder Schwefelatome vorkommen. Die Bindungsvalenz kann an einem beliebigen aromatischen Kohlenstoffatom oder an einem Stickstoffatom sein.

Ein monocyclischer Heteroarylring gemäß der vorliegenden Erfindung hat 5 oder 6 Ringatome.

Heteroarylringe mit 5 Ringatomen umfassen beispielsweise die Ringe:
Thienyl, Thiazolyl, Furanyl, Pyrrolyl, Oxazolyl, Imidazolyl, Pyrazolyl, Isoxazolyl, Isothiazolyl, Oxadiazolyl, Triazolyl, Tetrazolyl und Thiadiazolyl.

Heteroarylringe mit 6 Ringatomen umfassen beispielsweise die Ringe:
Pyridinyl, Pyridazinyl, Pyrimidinyl, Pyrazinyl und Triazinyl.

Ein bicyclischer Heteroarylring gemäß der vorliegenden Erfindung hat 9 bis 10 Ringatome.

Heteroarylringe mit 9 Ringatomen umfassen beispielsweise die Ringe:
Indolyl, Isoindolyl, Indazolyl, Benzothiazolyl, Benzofuranyl, Benzothiophenyl, Benzimidazolyl, Benzoxazolyl, Azocinyl, Indolizinyl, Purinyl.

Heteroarylringe mit 10 Ringatomen umfassen beispielsweise die Ringe:
Isochinolinyl, Chinolinyl, Cinnolinyl, Chinazolinyl, Chinoxalinyl, Phthalazinyl, 1,7- oder 1,8-Naphthyridinyl, Pteridinyl

Monocyclische Heteroarylringe mit 5 oder 6 Ringatomen sind bevorzugt.

Hydrierte bicyclische Aryl- oder Heteroarylringe sind Bizyklen, bei denen ein Ring teilweise oder vollständig hydriert vorliegt. Die Bindungsvalenz kann an einem beliebigen Atom des aromatischen Teiles des hydrierten bicyclischen Aryl- oder Heteroarylringes sein.

Hydrierte bicyclische Aryl- oder Heteroarylringe umfassen beispielsweise die Ringe: Indanyl, 1,2,3,4-Tetrahydro-naphthalenyl, 1,2,3,4-Tetrahydro-isochinolinyl, 1,2,3,4-Tetrahydro-chinolinyl, 2,3-Dihydro-1H-indolyl, 2,3-Dihydro-1H-isoindolyl, 4,5,6,7-Tetrahydro-thieno[2,3-c]pyridinyl, 4,5,6,7-Tetrahydro-thieno[3,2-c]pyridinyl, 2,3-Dihydro-benzofuranyl, Benzo[1,3]dioxolyl, 2,3-Dihydro-benzo[1,4]dioxinyl, Chromanyl, 1,2,3,4-Tetrahydro-chinoxalinyl, 3,4-Dihydro-2H-benzo[1,4]oxazinyl.

Hydrierte bicyclische Aryl- oder Heteroarylringe bei denen ein Ring teilweise oder vollständig hydriert vorliegt, können gegebenenfalls eine oder zwei Carbonylgruppen im Ringsystem enthalten.
Beispiele hierfür sind Indolonyl, Isoindolonyl, Benzoxazinonyl, Phthalazinonyl, Chinolonyl, Isochinolonyl, Phthalidyl, Thiophthalidyl.

### Heterocyclyl

Heterocyclyl im Sinne der Erfindung ist ein vollständig hydriertes Heteroaryl (vollständig hydriertes Heteroaryl = gesättigtes Heterocyclyl) d.h. ein nicht aromatisches Ringsystem mit mindestens einem von einem Kohlenstoff verschiedenen Heteroatom. Als Heteroatome können Stickstoffatome, Sauerstoffatome und/oder Schwefelatome vorkommen. Die Bindungsvalenz kann an einem beliebigen Kohlenstoffatom oder an einem Stickstoffatom sein.

Heterocycylring mit 3 Ringatomen umfasst beispielsweise:
Aziridinyl.

Heterocycylring mit 4 Ringatomen umfasst beispielsweise:
Azetidinyl, Oxetanyl.

Heterocycylringe mit 5 Ringatomen umfassen beispielsweise die Ringe:
Pyrrolidinyl, Imidazolidinyl, Pyrazolidinyl und Tetrahydrofuranyl.

Heterocyclylringe mit 6 Ringatomen umfassen beispielsweise die Ringe:
Piperidinyl, Piperazinyl, Morpholinyl, Tetrahydropyranyl und Thiomorpholinyl

Heterocycylringe mit 7 Ringatomen umfassen beispielsweise:
Azepanyl, Oxepanyl, [1,3]-Diazepanyl, [1,4]-Diazepanyl.

Heterocycylringe mit 8 Ringatomen umfassen beispielsweise:
Oxocanyl, Azocanyl.

Heterocyclylringe können gegebenenfalls teilweise ungesättigt sein und/oder auch eine Carbonylgruppe im Ring enthalten.
Beispiele hierfür sind Dihydro-furan-2-onyl, Pyrrolidin-2-onyl, Piperazin-2-onyl, Morpholin-2-onyl, 3(2H)-Pyridazinonyl, 5,6-Dihydro-2-pyran-2-onyl, 5,6-Dihydropyridin-2(1H)-onyl, 2-Piperidonyl, 1,2,3,6-Tetrahydropyridinyl.

### Halogen

Die Bezeichnung Halogen umfasst Fluor, Chlor, Brom und Iod.
Ebenfalls als von der vorliegenden Erfindung als erfasst anzusehen sind alle Verbindungen, die sich ergeben durch jede mögliche Kombination der oben genannten möglichen, bevorzugten und besonders bevorzugten Bedeutungen der Substituenten.

Besondere Ausführungsformen der Erfindung bestehen darüber hinaus in Verbindungen, die sich durch Kombination der direkt in den Beispielen offenbarten Bedeutungen für die Substituenten ergeben.

Ebenfalls als von der vorliegenden Erfindung als erfasst anzusehen sind die Salze der Verbindungen.

Die Formulierung der erfindungsgemäßen Verbindungen zu pharmazeutischen Präparaten erfolgt in an sich bekannter Weise, indem man den oder die Wirkstoffe mit den in der Galenik gebräuchlichen Hilfsstoffen in die gewünschte Applikationsform überführt.

Als Hilfsstoffe können dabei beispielsweise Trägersubstanzen, Füllstoffe, Sprengmittel, Bindemittel, Feuchthaltemittel, Gleitmittel, Ab- und Adsorptionsmittel, Verdünnungsmittel, Lösungsmittel, Cosolventien, Emulgatoren, Lösungsvermittler, Geschmackskorrigentien, Färbemittel, Konservierungs-, Stabilisierungs-, Netzmittel, Salze zur Veränderung des osmotischen Drucks oder Puffer zum Einsatz kommen. Dabei ist auf Remington's Pharmaceutical Science, 15th ed. Mack Publishing Company, East Pennsylvania (1980) hinzuweisen.

Die pharmazeutischen Formulierungen können
in fester Form, zum Beispiel als Tabletten, Dragees, Pillen, Suppositorien, Kapseln, transdermale Systeme oder
in halbfester Form , zum Beispiel als Salben, Cremes, Gele, Suppositiorien, Emulsionen oder
in flüssiger Form, zum Beispiel als Lösungen, Tinkturen, Suspensionen oder Emulsionen vorliegen.

Hilfsstoffe im Sinne der Erfindung können beispielsweise Salze, Saccharide (Mono-, Di-, Tri-, Oligo-, und/oder Polysaccharide), Proteine, Aminosäuren, Peptide, Fette, Wachse, Öle, Kohlenwasserstoffe sowie deren Derivate sein, wobei die Hilfsstoffe natürlichen Ursprungs sein können oder synthetisch bzw. partial synthetisch gewonnen werden können.

Für die orale oder perorale Applikation kommen insbesondere Tabletten, Dragees, Kapseln, Pillen, Pulver, Granulate, Pastillen, Suspensionen, Emulsionen oder Lösungen in Frage.
Für die parenterale Applikation kommen insbesondere Suspensionen, Emulsionen und vor allem Lösungen in Frage.

Aufgrund ihrer anti-entzündlichen und zusätzlichen immunsuppressiven Wirkung können die erfindungsgemäßen Verbindungen der allgemeinen Formel (I) als Medikamente zur Behandlung oder Prophylaxe folgender Krankheitszustände bei Säugetieren und Menschen, für die lokale und systemische Applikation Verwendung finden:
(i) Lungenerkrankungen, die mit entzündlichen, allergischen und / oder proliferativen Prozessen einhergehen:
   - Chronisch obstruktive Lungenerkrankungen jeglicher Genese, vor allem Asthma bronchiale
   - Bronchitis unterschiedlicher Genese
   - Adult respiratory distress syndrome (ARDS), akutes Atemnotssyndrom
   - Bronchiektasen
   - Alle Formen der restriktiven Lungenerkrankungen, vor allem allergische Alveolitis,
   - Lungenödem, insbesondere allergisches
   - Sarkoidosen und Granulomatosen, insbesondere Morbus Boeck
(ii) Rheumatische Erkrankungen / Autoimmunerkrankungen / Gelenkerkrankungen, die mit entzündlichen, allergischen und / oder proliferativen Prozessen einhergehen:
   - Alle Formen rheumatischer Erkrankungen, insbesondere rheumatoide Arthritis, akutes rheumatisches Fieber, Polymyalgia rheumatica, Morbus Behcet
   - Reaktive Arthritis
   - Entzündliche Weichteilerkrankungen sonstiger Genese
   - Arthritische Symtome bei degenerativen Gelenkerkrankungen (Arthrosen)
   - Vitiligo
   - Kollagenosen jeglicher Genese, z.B. systemischer Lupus erythematodes, Sklerodermie, Polymyositis, Dermatomyositis- Sjögren-Syndrom, Still-Syndrom, Felty-Syndrom
   - Sarkoidosen und Granulomatosen
   - Weichteilrheumatismus
(iii) Allergien oder pseudoallergische Erkrankungen, die mit entzündlichen, und /oder proliferativen Prozessen einhergehen:
   - alle Formen allergischer Reaktionen, z.B. Quincke Ödem, Heuschnupfen, Insektenstich, allergische Reaktionen auf Arzneimittel, Blutderivate, Kontrastmittel etc., Anaphylaktischer Schock, Urtikaria, allergische und irritative Kontakdermatitis, allergische Gefäßerkrankungen
   - Vasculitis allergica
(iv) Gefäßentzündungen (Vaskulitiden)
   - Panarteriitis nodosa, Arteriitis temporalis, Erythema nodosum
   - Polyarteritis nodosa
   - Wegner Granulomatose
   - Riesenzellarteritis
(v) Dermatologische Erkrankungen, die mit entzündlichen, allergischen und / oder proliferativen Prozessen einhergehen:
   - Atopische Dermatitis (vor allem bei Kindern)
   - Sämtliche Ekzemformen wie z.B. atopisches Ekzem (v.a. bei Kindern)
   - Exantheme jedweder Genese oder Dermatosen
   - Psoriasis und Parapsoriasis-Formenkreis
   - Pityriasis rubra pilaris
   - Erythematöse Erkrankungen, ausgelöst durch unterschiedlichen Noxen, z.B. Strahlen, Chemikalien, Verbrennungen etc.
   - Bullöse Dermatosen wie z.B. autoimmuner Pemphigus vulgaris, bullöses Pemphigoid
   - Erkrankungen des lichenoiden Formenkreises,
   - Pruritus (z. B. allergischer Genese)
   - Rosacea-Formenkreis
   - Erythema exsudativum multiforme
   - Manifestation von Gefäßerkrankungen
   - Haarausfall wie Alopecia areata
   - Kutane Lymphome
(vi) Nierenerkrankungen, die mit entzündlichen, allergischen und / oder proliferativen Prozessen einhergehen:
   - Nephrotisches Syndrom
   - Alle Nephritiden, z.B. Glomerulonephritis
(vii) Lebererkrankungen, die mit entzündlichen, allergischen und / oder proliferativen Prozessen einhergehen:
   - akute Hepatitis unterschiedlicher Genese
   - chronisch aggressive und / oder chronisch intermittierende Hepatitis
(viii) Gastrointestinale Erkrankungen, die mit entzündlichen, allergischen und / oder proliferativen Prozessen einhergehen:
   - regionale Enteritis (Morbus Crohn)
   - Colitis Ulcerosa
   - Gastroenteritiden anderer Genese, z.B. einheimische Sprue
(ix) Augenerkrankungen, die mit entzündlichen, allergischen und / oder proliferativen Prozessen einhergehen:
   - allergische Keratitis, Uveitis, Iritis,
   - Konjunktivitis
   - Blepharitis
   - Neuritis nervi optici
   - Chorioiditis
   - Ophthalmia sympathica
(x) Erkrankungen des Hals-Nasen-Ohren-Bereiches, die mit entzündlichen, allergischen und / oder proliferativen Prozessen einhergehen:
   - allergische Rhinitis, Heuschnupfen
   - Otitis extema, z.B. bedingt durch Kontaktekzem
(xi) Neurologische Erkrankungen, die mit entzündlichen, allergischen und / oder proliferativen Prozessen einhergehen:
   - Himödem, vor allem allergisches Himödem
   - Multiple Sklerose
   - akute Encephalomyelitis
   - Meningitis, vor allem allergische
   - Guillain-Barre Syndrom
   - Morbus Alzheimer
(xii) Bluterkrankungen, die mit entzündlichen, allergischen und / oder proliferativen Prozessen einhergehen wie z. B.: M. Hodgkin oder Non-Hodgkin Lymphome, Thrombozytaemien, Erythrozytosen
   - Erworbene hämolytische Anämie
   - Idiopathische Thrombozytopenie
   - Idiopathische Granulozytopenie
(xiii) Tumorerkrankungen, die mit entzündlichen, allergischen und / oder proliferativen Prozessen einhergehen
   - Akute lymphatische Leukämie
   - Maligne Lymphome
   - Lymphogranulomatosen
   - Lymphosarkome
(xiv) Endokrine Erkrankungen, die mit entzündlichen, allergischen und / oder proliferativen Prozessen einhergehen wie z. B.:
   - Endokrine Orbitopathie
   - Thyreoiditis de Quervain
   - Hashimoto Thyreoiditis
   - Morbus Basedow
   - Granulomatous thyroiditis
   - Struma lymphomatosa
   - Autoimmune adrenalitis
   - Diabetes mellitus, insbesondere Typ 1 Diabetes
   - Endometriose
(xv) Organ- und Gewebstransplantationen, Graft-versus-host-disease
(xvi) Schwere Schockzustände, z.B anaphylaktischer Schock, systemic inflammatory response syndrome (SIRS)

Ein Gegenstand der Erfindung ist die Verwendung der erfindungsgemäßen Verbindungen der allgemeinen Formel (I) zur Herstellung eines Arzneimittels.

Ein weiterer Gegenstand der Erfindung ist die Verwendung der erfindungsgemäßen Verbindungen zur Behandlung von Erkrankungen, die mit entzündlichen, allergischen und / oder proliferativen Prozessen einhergehen.

In der allgemeinen Formel (I) kann Q₁ stehen für einen Heteroarylring mit 5 Ringatomen.

Bevorzugt steht Q₁ für einen Pyrazolyl-, Thienyl-, Imidazolyl- oder 1,2,4-Oxadiazolylring

Besonders bevorzugt steht Q₁ für einen Pyrazolyl- oder Thienylring.

In der allgemeinen Formel (I) können R¹ und R² unabhängig voneinander stehen für:
(i) Wasserstoff, Hydroxy, Nitro, Halogen, Cyano, -CF₃, -NR⁵R⁶ oder
(ii) -C(O)-OR¹⁰, -C(O)-R⁷, -C(O)-C₁-C₃-Fluoralkyl, -C(O)-NR⁵R⁶, -NH-C(O)-R⁷ oder
(iii) einen C₁-C₆-Alkyl-, C₂-C₆ -Alkenyl-, C₂-C₆-Alkinyl-, C₁-C₆-Alkoxy-, C₁-C₆-Fluoralkyl- oder einen C₁-C₆-Fluoralkoxyrest,
   jeweils gegebenenfalls mit -C₁-C₃-Alkoxy, Hydroxy, -C(O)-OR¹⁰ oder -NR⁸R⁹ ein- oder mehrfach, gleich oder verschieden substituiert, oder
(iv) -C-SO₂-NR⁵R⁶, -SO₂-R⁷, -SO₂-NR⁵R⁶.

Bevorzugt stehen R¹ und R² unabhängig voneinander für:
(i) Wasserstoff, Hydroxy, Halogen, Cyano, -CF₃, -NR⁵R⁶ oder
(ii) einen C₁-C₆-Alkyl-, C₁-C₆-Alkoxy-, C₁-C₆-Fluoralkyl- oder einen C₁-C₆-Fluoralkoxyrest oder
(iii) -SO₂-R⁷,

Besonders bevorzugt stehen R¹ und R² unabhängig voneinander für:
(i) Wasserstoff oder
(ii) einen C₁-C₆-Alkylrest.

In der allgemeinen Formel (I) kann Q₂ stehen für einen Heteroaryl- oder einen hydrierten bizyklischen Heteroarylring.

Bevorzugt steht Q₂ für einen:
Thienyl-, Thiazolyl-, Furanyl-, Pyrrolyl-, Oxazolyl-, Imidazolyl-, Pyrazolyl-, Isoxazolyl, Isothiazolyl, 1,2,3-Triazolyl, Pyridinyl-, Pyridazinyl-, Pyrimidinyl-, Pyrazinyl- , 1,2,3,4-Tetrahydro-isochinolinyl-, 1,2,3,4-Tetrahydro-chinolinyl-, 2,3-Dihydro-1H-indolyl-, 2,3-Dihydro-1H-isoindolyl-, 4,5,6,7-Tetrahydro-thieno[2,3-c]pyridinyl-, 4,5,6,7-Tetrahydro-thieno[3,2-c]pyridinyl-, 2,3-Dihydro-benzofuranyl-, Benzo[1,3]dioxolyl-, 2,3-Dihydro-benzo[1,4]dioxinyl-, 1,2,3,4-Tetrahydro-chinoxalinyl - oder einen 3,4-Dihydro-2H-benzo[1,4]oxazinylring.

Besonders bevorzugt steht Q₂ für einen Pyrazolylring.

In der allgemeinen Formel (I) können R³ und R⁴ unabhängig voneinander stehen für:
(i) Wasserstoff, Halogen oder -NR¹¹R¹², oder
(ii) einen C₁-C₆-Alkyl- oder C₁-C₆-Alkoxyrest, die jeweils gegebenenfalls ein- oder mehrfach, gleich oder verschieden mit Hydroxy, C₁-C₃-Alkoxy, -NR⁸R⁹ oder Heterocyclyl substituiert sind, wobei der Heterocylylring ein- oder mehrfach, gleich oder verschieden substituiert sein kann mit C₁-C₃-Alkyl oder -C(O)-R⁷.

Bevorzugt stehen R³ und R⁴ unabhängig voneinander für:
(i) Wasserstoff, Halogen, -NR¹¹R¹² oder
(ii) einen C₁-C₆-Alkyl- oder C₁-C₆-Alkoxyrest,
   jeweils gegebenenfalls mit Morpholin oder -NR⁸R⁹ substituiert.

Besonders bevorzugt stehen R³ und R⁴ unabhängig voneinander für Wasserstoff oder C₁-C₆-Alkyl.

In der allgemeinen Formel (I) können R⁵ und R⁶ unabhängig voneinander stehen für: für Wasserstoff oder einen C₁-C₆ Alkylrest, der gegebenenfalls ein- oder mehrfach, gleich oder verschieden mit Hydroxy, -C₁-C₃-Alkoxy, -NR⁸R⁹ oder Heterocyclyl substituiert ist, wobei der Heterocylylring ein- oder mehrfach, gleich oder verschieden substituiert sein kann mit C₁-C₃-Alkyl oder -C(O)-R⁷,
oder
- R⁵ und R⁶: bilden alternativ gemeinsam mit dem Stickstoffatom einen 5- bis 7-gliedrigen Ring, der gegebenenfalls zusätzlich zum Stickstoffatom ein weiteres Heteroatom enthält und der gegebenenfalls mit C₁-C₆-Alkyl und/oder mit -C(O)-R⁷ ein- oder mehrfach, gleich oder verschieden substituiert ist.

Bevorzugt stehen R⁵ und R⁶ unabhängig voneinander für Wasserstoff oder einen C₁-C₆ Alkylrest, der gegebenenfalls ein- oder mehrfach, gleich oder verschieden mit Hydroxy, -NR⁸R⁹, Halogen oder C₁-C₃-Alkoxy substituiert sein kann.

Besonders bevorzugt stehen R⁵ und R⁶ unabhängig voneinander für Wasserstoff oder einen C₁-C₄-Alkylrest.

In der allgemeinen Formel (I) kann R⁷ stehen für einen C₁-C₆ Alkylrest.

Bevorzugt steht R⁷ für einen C₁-C₄-Alkylrest.

Besonders bevorzugt steht R⁷ für einen C₁-C₃-Alkylrest.

In der allgemeinen Formel (I) können R⁸, R⁹, R¹⁰ unabhängig voneinander stehen für Wasserstoff oder einen C₁-C₆-Alkylrest.

Bevorzugt stehen R⁸ und R⁹ unabhängig voneinander für Wasserstoff oder einen C₁-C₄-Alkylrest und R¹⁰ für Wasserstoff.

Besonders bevorzugt stehen R⁸ und R⁹ unabhängig voneinander für Wasserstoff oder einen C₁-C₃-Alkylrest und R¹⁰ für Wasserstoff.

In der allgemeinen Formel (I) können R¹¹ und R¹² unabhängig voneinander stehen für: Wasserstoff oder einen C₁-C₆ Alkylrest, der gegebenenfalls ein- oder mehrfach, gleich oder verschieden mit Hydroxy, -C₁-C₃-Alkoxy, -NR⁸R⁹ oder Heterocyclyl substituiert ist, wobei der Heterocylylring ein- oder mehrfach, gleich oder verschieden substituiert sein kann mit C₁-C₃-Alkyl oder -C(O)-R⁷.

Bevorzugt stehen R¹¹ und R¹² unabhängig voneinander für Wasserstoff oder einen C₁-C₆ Alkylrest, der gegebenenfalls ein- oder mehrfach, gleich oder verschieden mit Morpholin oder durch -N(CH₃)₂, -NH-CH₃ oder -NH-C₂H₅ substituiert sein kann.

Eine bevorzugte Untergruppe bilden Verbindungen der Formel (I) mit den Bausteinen A, B, C und D,
in der die Bausteine B und D in ortho-Stellung zueinander stehen und
- Q₁: für einen einen Pyrazolyl-, Thienyl-, Imidazolyl- oder 1,2,4-Oxadiazolylring steht,
- R¹ und R²: unabhängig voneinander stehen für
- (i): Wasserstoff, Hydroxy, Halogen, Cyano, -CF₃, -NR⁵R⁶ oder
- (ii): einen C₁-C₆-Alkyl-, C₁-C₆-Alkoxy-, C₁-C₆-Fluoralkyl- oder einen C₁-C₆-Fluoralkoxyrest oder
- (iii): -SO₂-R⁷,
- Q₂: für einen Thienyl-, Thiazolyl-, Furanyl-, Pyrrolyl-, Oxazolyl-, Imidazolyl-, Pyrazolyl-, Isoxazolyl, Isothiazolyl, 1,2,3-Triazolyl, Pyridinyl- , Pyridazinyl-, Pyrimidinyl-, Pyrazinyl-, 1,2,3,4-Tetrahydro-isochinolinyl-, 1,2,3,4-Tetrahydro-chinolinyl-, 2,3-Dihydro-1H-indolyl-, 2,3-Dihydro-1H-isoindolyl-, 4,5,6,7-Tetrahydro-thieno[2,3-c]pyridinyl-, 4,5,6,7-Tetrahydro-thieno[3,2-c]pyridinyl-, 2,3-Dihydro-benzofuranyl-, Benzo[1,3]dioxolyl-, 2,3-Dihydro-benzo[1,4]dioxinyl-, 1,2,3,4-Tetrahydro-chinoxalinyl- oder einen 3,4-Dihydro-2H-benzo[1,4]oxazinylring steht,
- R³ und R⁴: unabhängig voneinander stehen für
- (i): Wasserstoff, Halogen, -NR¹¹R¹² oder
- (ii): einen C₁-C₆-Alkyl- oder C₁-C₆-Alkoxyrest, lig id="I20.1036" xbd="1992" xhg="575" ybd="3126" yhg="3065"/>jeweils gegebenenfalls mit Morpholin oder -NR⁸R⁹ substituiert, wobei
- R⁵ und R⁶: unabhängig voneinander stehen für Wasserstoff oder einen C₁-C₆ Alkylrest, der gegebenenfalls ein- oder mehrfach, gleich oder verschieden mit Hydroxy, -NR⁸R⁹, Halogen oder C₁-C₃-Alkoxy substituiert sein kann, und
- R⁷: für einen C₁-C₄ Alkylrest steht, und
- R⁸ und R⁹: unabhängig voneinander stehen für Wasserstoff oder einen C₁-C₄ Alkylrest,
- R¹¹ und R¹²: unabhängig voneinander stehen für Wasserstoff oder einen C₁-C₆ Alkylrest, der gegebenenfalls ein- oder mehrfach, gleich oder verschieden mit Morpholin oder durch -N(CH₃)₂, -NH-CH₃ oder -NH-C₂H₅ substituiert sein kann,
sowie deren Salze, Enantiomere und Diastereomere.

Besonders bevorzugt sind Verbindungen der allgemeinen Formel (I) mit den Bausteinen A, B, C und D,
in der die Bausteine B und D in ortho-Stellung zueinander stehen und
- Q₁: für einen Pyrazolyl- oder Thienylring steht, und
- R¹ und R²: unabhängig voneinander stehen für
- (i): Wasserstoff
- (ii): einen C₁-C₆-Alkylrest
- Q₂: für einen Pyrazolylring steht, und
- R³ und R⁴: unabhängig voneinander stehen für Wasserstoff oder einen C₁-C₆-Alkyl,
sowie deren Salze, Enantiomere und Diastereomere.

### Herstellung der erfindungsgemäßen Verbindungen

### a) Herstellung von Intermediaten der Formel (III)

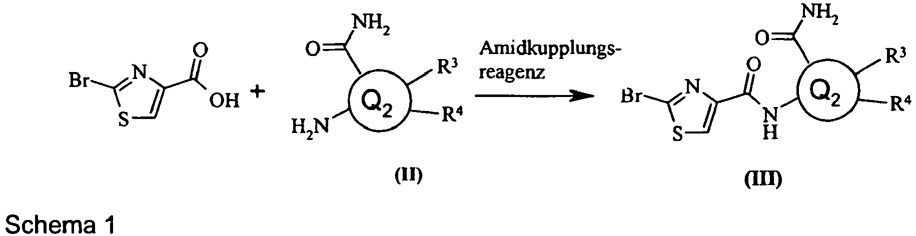

2-Bromthiazol-4-carbonsäure wird mit Intermediaten der Formel (II) durch ein Amidkupplungsreagenz (siehe Publikation Chemfiles, Peptide Synthesis, 2007, 7, Nr. 2 der Firma Sigma-Aldrich) zu Intermediaten der Formel (III) umgesetzt. Hierbei können zum Beispiel Carbodiimide (zum Beispiel N-Cyclohexyl-N'-(2-morpholinoethyl)carbodiimid metho-p-toluolsulfonat CAS2491-17-0) oder Uroniumsalze (zum Beispiel O-(7-Azabenzotriazol-1-yl)-*N,N,N'N'-*tetramethyluroniumhexafluorophosphat (HATU)) verwendet werden in Kombination mit einer Base wie zum Beispiel Pyridin.

### b) Herstellung des Endproduktes

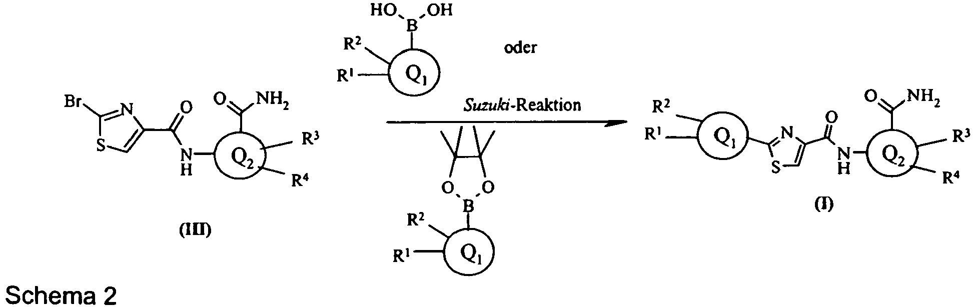

Intermediate der Formel (III) werden mit Boronsäuren oder Boronsäurepinakolestern im Rahmen **einer** *Suzuki-Miyaura-Reaktion* zu den erfindungsgemäßen Verbindungen der Formel (I) umgesetzt. Als Katalysatoren bzw. Liganden kommen dabei zum Beispiel die in N. Miyaura, A. Suzuki, Chem. Rev.; 1995; 95(7); 2457-2483 oder in C. J. O'Brien et. al. Chem. Eur. J. 2006, 12, 4743 - 4748 sowie die in der Publikation Chemfiles, Catalysis, 2007, 7, Nr. 5 von der Firma Sigma-Aldrich beschrieben Katalysatoren bzw. Katalysatorensysteme in Betracht.
Hierbei haben Q₁, Q₂, R¹, R², R³ und R⁴ die in der allgemeinen Formel (I) gemäß der Ansprüche 1 bis 7 angegebenen Bedeutungen.

### Reinigung der erfindungsgemäßen Verbindungen

In einigen Fällen können die erfindungsgemäßen Verbindungen durch präparative HPLC zum Beispiel durch ein Autopurifier-Gerät der Firma Waters (Detektion der Verbindungen durch UV-Detektion sowie Elektrospray-Ionisation) in Kombination mit kommerziell erhältlichen, vorgepackten HPLC Säulen (zum Beispiel Säule XBridge (Firma Waters), C18, 5µm, 30 x 100mm) gereinigt werden. Als Lösemittelsystem kann Acetonitril/Wasser +0,1 % Trifluoressigsäure verwendet werden (Fluss 50 ml /min). Zum Entfernen des HPLC-Lösemittelgemisches kann eine Gefriertrocknung oder eine Zentrifugation erfolgen. Die so erhaltenen Verbindungen können als Trifluoressigsäure (TFA)-Salze vorliegen und können zu den jeweiligen freien Basen durch dem Fachmann bekannte Standard-Laborprozeduren überführt werden.
In einigen Fällen können die erfindungsgemäßen Verbindungen durch Chromatographie an Kieselgel gereinigt werden. Hierbei können zum Beispiel vorgepackte Silica Gel Cartridges (zum Beispiel von der Firma Separtis, *Isolute*® *Flash silica gel)* in Kombination mit dem Flashmaster II Chromatograhiegerät (Argonaut/Biotage) und Chromatographielösemittel bzw. -Gemische wie zum Beispiel Hexan, Essigester sowie Dichlormethan und Methanol in Betracht.

### Strukturanalytik der erfindungsgemäßen Verbindungen:

In einigen Fällen werden die erfindungsgemäßen Verbindungen durch LC-MS analysiert: Retentionszeiten Rₜ aus der LC-MS-Analytik: Detektion: UV = 200 - 400 nm (*Acquity* HPLC-System der Firma *Waters)*/ MS 100-800 Daltons; 20 V (Micromass / *Waters* ZQ 4000) im ESI⁺-Modus (zur Erzeugung positiv geladener Molekülionen); HPLC-Säute: X Bridge *(Waters),* 2.1 x 50 mm, BEH 1.7 µm; Flussmittel: A: H₂O/0.05% HC(O)OH, B: CH3CN/0.05% HC(O)OH. Gradient: 10-90% B in 1.7 min, 90% B für 0.2 min, 98-2% B in 0.6 min; Flussrate: 1.3 ml / min. Die Angabe *LC-MS (ZQ)* bezieht sich auf die Verwendung eines *Waters* ZQ4000 Gerätes und die Angabe *LC-MS (SQD)* auf die Benutzung eines Single Quadrupol API (Atomic Pressure lonization) Massendetektors (*Waters*) zur Aufnahme eines Massenspektrums.

### Beispiel 1

### 2-(1H-Pyrazol-4-yl)-thiazol-4-carbonsäure (3-carbamoyl-1-methyl-1 H-pyrazol-4-yl)-amid

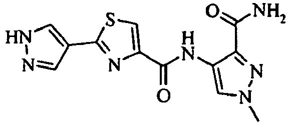

### a) Herstellung des Intermediates III.1

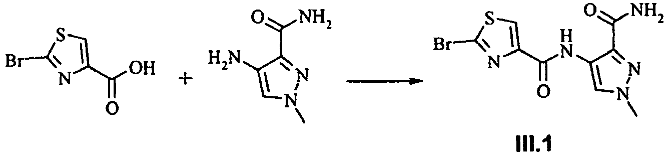

Eine Mischung aus 2-Bromthiazol-4-carbonsäure (891 mg), 4-Amino-1-methyl-1 H-pyrazol-3-carbonsäureamid (600 mg) und HATU (1.95 g) in DMF (5 ml) und Ethyldiisopropylamin (1.5 ml) wird über 20 h bei Raumtemperatur gerührt und dann auf Eiswasser gegossen. Der ausgefallene Feststoff wird abfiltriert, zweimal mit Wasser und je dreimal mit Diethylether und Essigester gewaschen und im Vakuum getrocknet (889 mg).
C₉H₈BrN₅O₂S (329.0), LC-MS (ZQ): Rₜ = 0.87, m/z = 330 [M+H]⁺. 1H-NMR (300 MHz, D6-DMSO): 3.88 (s, 3H), 7.45 (br. s., 1 H), 7.70 (br. s., 1 H), 8.31 (s, 1 H), 8.41 (s, 1H), 11.0 (s, 1H).

### b) Herstellung des Endproduktes

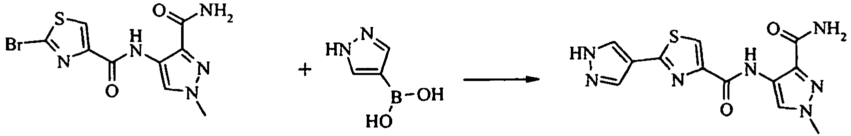

Intermediat III.1 (150 mg) und 1H-Pyrazol-4-boronsäure (152 mg) werden mit THF (3 ml) und Kaliumcarbonatlösung (wässrig, 1 M, 2 ml) versetzt und danach gibt man 1,1'-Bis(diphenylphosphino)ferrocen-palladium-dichlorid Dichlormethan Komplex (74 mg) zu und erhitzt 45 min in einer Microwelle bei 130°C (Gerät CEM Explorer von der Firma CEM, 300 Watt). Man führt eine wässrige Aufarbeitung mit Essigester und Wasser durch, trocknet die organische Phase mit Natriumsulfat, engt ein und reinigt den Rückstand durch HPLC. Man erhält 4.5 mg eines weißen Schaumes.

### Bedingungen für die präparative Reinigung:

| | |
|---|---|
| Säule: | XBridge (Firma Waters) C18 5µ 150×30 mm |
| Lösemittel A: | H₂O / 0.1 % HC(O)OH |
| Lösemittel B: | Acetonitril |

| | | |
|---|---|---|
| Gradient: | 0 min | 90%A 10%B |
| | 1.00 min | 90%A 10%B |
| | 7.50 min | 60%A 40%B |
| | 7.52 min | 1%A 99%B |
| | 10.00 min | 1%A 99%B |

| | |
|---|---|
| Flussrate: | 50.0 mUmin |
| Injektionsvolumen: | 1 × 1.5 mL |
| Detektion: | DAD (200-400 nm) TAC ; MS-ESI+ (m/z=160-1000 m/z) TIC |
| Temperatur: | Raumtemperatur. |

### Bedingungen für die HPLC-Analytik:

| | |
|---|---|
| Säule: | Acquity BEH C18 1.7µm 50×2.1 mm |
| Lösemittel A: | H₂O / 0.05% HC(O)OH |
| Lösemittel B: | Acetonitril |

| | | |
|---|---|---|
| Gradient: | 0 min | 99%A 1%B |
| | 1.6 min | 1%A 99%B |
| | 2.0 min | 1%A 99%B |

| | |
|---|---|
| Flussrate: | 0.8 mL/min |
| Injektionsvolumen: | 2.0 µl |
| Detektion: | DAD (200-400 nm) TAC ; MS-ESI+,ESI- (120-1000 m/z) TIC |
| Temperatur: | 60°C |

C₁₂H₁₁N₇O₂S (317.3), Rₜ = 0.71, m/z = 318.2 [M+H]⁺.

### Beispiel 2

### N-[2-(Aminocarbonyl)phenyl]-2-(5-methyl-3-thienyl)-4-thiazolcarboxamid

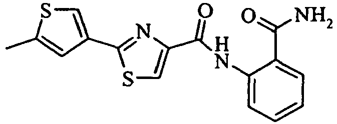

### a) Herstellung des Intermediates III.2

### N-[2-(Aminocarbonyl)phenyl]-2-brom-4-thiazolcarboxamid

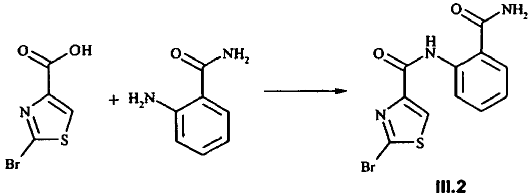

2-Brom-4-thiazolcarbonsäure (1 g), HATU (2.01 g) und 2-Aminobenzamid (0.65 g) in N,N-Dimethylformamid (DMF) (20 ml) werden vorgelegt. Man kühlt mit einem Eisbad ab und gibt N,N-Diisopropylethylamin (0.90 ml) zu. Man lässt 6 Tage bei Raumtemperatur rühren, gießt das Reaktionsgemisch auf Eiswasser, lässt unter Rühren auftauen und saugt den ausgefallenen Feststoff ab, wäscht zweimal mit Wasser, zweimal mit Diethylether und trocknet im Vakuum. Man erhält das Intermediat III.2 als Feststoff (1.4 g).
C₁₁H₈BrN₃O₂S, M = 326.2. 1 H-NMR (300 MHz, D6-DMSO): δ = 7.20 (m, 1 H), 7.56 (m, 1 H), 7.79 (s, 1 H), 7.84 (m, 1 H), 8.30 (s, 1 H), 8.47 (m, 1 H), 8.67 (m, 1 H), 12.9 (s, 1 H).

### b) Herstellung des Endproduktes

N-[2-(Aminocarbonyl)phenyl]-2-(5-methyl-3-thienyl)-4-thiazolcarboxamid wird mit Hilfe der Suzuki-Reaktion (analog zu A. Suzuki et. al. J. Am. Chem. Soc. 1989, 111, 314.) hergestellt.

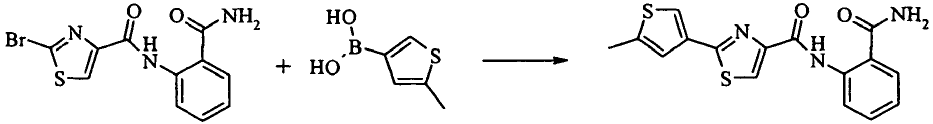

Man legt das Intermediat III.2 (65 mg) und 2-Methyl-4-thiophenboronsäure (40 mg) in Toluol (1.5 ml), Ethanol (1.5 ml) und wässriger Natriumcarbonat-Lösung (2 M, 180 Microliter) vor, gibt Pd(PPh₃)₄ (23 mg) zu und erhitzt in der Mikrowelle 15 min bei 120°C (300W) (Gerät CEM Explorer). Das Reaktionsgemisch wird mit Wasser verdünnt und mit Essigester extrahiert. Die organische Phase wird eingeengt und der Rückstand durch HPLC gereinigt. Man erhält Beispiel 2 (35 mg) als Feststoff.
C₁₆H₁₃N₃O₂S₂, M = 343.4, LC-MS (ZQ): Rₜ = 1.20, m/z = 344 [M+H]⁺.

### Beispiel 3

### N-[2-(Aminocarbonyl)-5-methylphenyl)-2-(1H-pyrazol-4-yl)-thiazol-4-carbonsäureamid

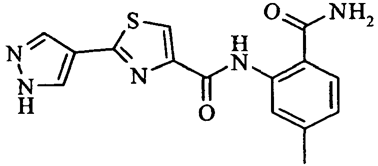

### a) Herstellung des Intermediates III.3

### N-[2-(Aminocarbonyl)-5-methylphenyl]-2-brom-4-thiazolcarboxamid

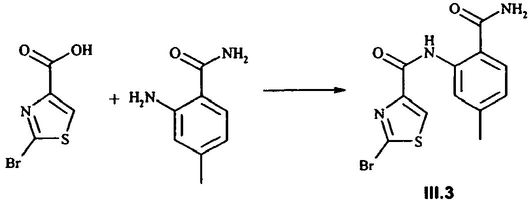

Analog zur Synthese von Intermediat III.2 wird 2-Brom-4-thiazolcarbonsäure und 2-Amino-4-methylbenzamid zu N-[2-(Aminocarbonyl)-5-methylphenyl]-2-brom-4-thiazolcarboxamid (Intermediat III.3) umgesetzt.
C₁₂H₁₀BrN₃O₂S, M = 339.0, LC-MS (ZQ): Rₜ = 1.04, m/z = 340 [M+H]⁺. 1 H-NMR (300 MHz, D6-DMSO): δ = 2.32 (s, 3H), 6.97 (m, 1 H) 7.65 (br. s., 1 H), 7.70 (d, 1 H), 8.18 (br. s., 1H), 8.40 (s, 1 H), 8.49 (m, 1 H), 13.0 (s, 1 H).

### b) Herstellung des Endproduktes

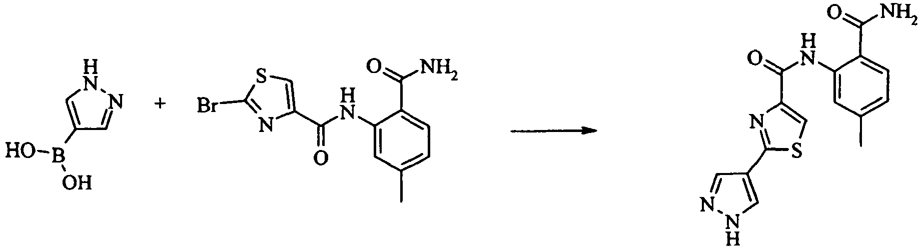

Eine Mischung aus Intermediat III.3 (150 mg), 1 H-Pyrazol-4-boronsäure (63 mg) in Ethylenglycoldimethylether (3 ml) und wässriger Natriumhydrogencarbonatlösung (2 ml, 2M) wird in Gegenwart von Pd(PPh₃)₄ (55 mg) in einer Microwelle (CEM Explorer, 300 Watt) bei 110°C 40 min lang erhitzt. Nach Aufarbeitung wie bei Beispiel 2 erhält man N-[2-(Aminocarbonyl)-5-methylphenyl]-2-(1H-pyrazol-4-yl)-thiazol-4-carbonsäureamid als Feststoff (4.9 mg).
C₁₅H₁₃N₅O₂S, M = 327.1, LC-MS (ZQ): Rₜ = 0.99, m/z = 328 [M+H]⁺.

### TLR induzierte Zytokinfreisetzung in humanen "peripheral blood mononuclear cells" (PBMC)

### Versuchsprinzip

Aus humanem Vollblut isolierte PBMCs werden mit einem TLR Liganden stimuliert. Die Zytokin-Bestimmung wird mittels ELISA-Kits, eine Proliferations- /Zellstoffwechselbestimmung wird mit Alamarblue durchgeführt.

### PBMC-Isolierung:

Für die Zellpräparation werden ca. 200 ml Blut mit einem Antikoagulanz (z.B. Citrat-Monovetten) versetzt. Pro Leucosep-Röhrchen werden 15ml Histopaque (Raumtemperatur, RT) eingefüllt und durch kurzes Anzentrifugieren (eine Minute bei 1000 x g, RT) durch die eingesetzte Fritte nach unten gedrückt. In die so vorbereiteten Röhrchen werden 20ml Blut gegeben und für 15 Minuten bei 800xg (RT) zentrifugiert. Nach der Zentrifugation ergibt sich von oben nach unten folgende Schichtung:
Plasma - PBMC - Histopaque - Filterscheibe - Histopaque - Erythrozyten und Granulozyten. Das überstehende Plasma wird abgesaugt. Die PBMC werden mitsamt dem darunterliegenden Histopaque in ein neues 50ml Röhrchen überführt, wobei immer der Inhalt von zwei Leucosep-Röhrchen
in ein 50ml Röhrchen gegeben wird. Die 50ml Röhrchen werden dann mit PBS auf 50ml aufgefüllt. Diese Zellsuspension wird für zehn Minuten bei 300xg (RT) zentrifugiert.
Der flüssige Überstand wird abgekippt und das Zellpellet mit wenig PBS resuspendiert und anschließend mit PBS auf 50ml aufgefüllt. Dieser Waschschritt wird zweimal wiederholt. Das entstandene Pellet wird in einem definierten Volumen von Medium (mit Zusätzen) aufgenommen. Zur Testung der Substanzen werden PBMC mit titrierten Konzentrationen der Testsubstanzen z.B. in Gegenwart oder Abwesenheit von TLR7 oder TLR9 Liganden für 18 Stunden inkubiert. Am nächsten Tag werden die Überstände mittels spezifischer ELISA auf den Gehalt von TNF-alpha oder anderen Zyto- oder Chemokinen untersucht. Die Stoffwechselaktivität der behandelten Zellen wird mithilfe von Alamarblue bestimmt.

### Ergebnisse:

| **Beispiel** | **EC₅₀ (TNF-α TLR7)** |
|---|---|
| 1 | 5.0e-8 mol/L |
| 2 | 3.8e-6 mol/L |
| 3 | 1.2e-7 mol/L |

## Patentansprüche

1. Verbindungen der allgemeinen Formel (I) mit den Bausteinen A, B, C und D , 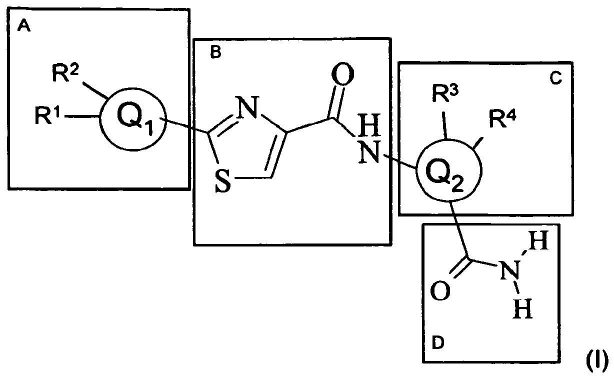 in der
die Bausteine B und D in ortho-Stellung zueinander stehen und
Q₁ für einen Heteroarylring mit 5 Ringatomen steht,
R¹ und R² unabhängig voneinander stehen für
(i) Wasserstoff, Hydroxy, Nitro, Halogen, Cyano, -CF₃, -NR⁵R⁶ oder
(ii) -C(O)-OR¹⁰, -C(O)-R⁷, -C(O)-C₁-C₃-Fluoralkyl,
-C(O)-NR⁵R⁶, -NH-C(O)-R⁷ oder
(iii) einen C₁-C₆-Alkyl-, C₂-C₆ -Alkenyl-, C₂-C₆-Alkinyl-, C₁-C₆-Alkoxy-, C₁-C₆-Fluoralkyl- oder einen C₁-C₆-Fluoralkoxyrest,
jeweils gegebenenfalls mit -C₁-C₃-Alkoxy, Hydroxy, -C(O)-OR¹⁰ oder -NR⁸R⁹ ein- oder mehrfach, gleich oder verschieden substituiert, oder
(iv) -O-SO₂-NR⁵R⁶, -SO₂-R⁷, -SO₂-NR⁵R⁶ ,
Q₂ für einen Heteroaryl- oder einen hydrierten bizyklischen Heteroarylring steht,
R³ und R⁴ unabhängig voneinander stehen für
(i) Wasserstoff, Halogen oder -NR¹¹R¹², oder
(ii) einen C₁-C₆-Alkyl- oder C₁-C₆-Alkoxyrest, die jeweils gegebenenfalls ein- oder mehrfach, gleich oder verschieden mit Hydroxy, C₁-C₃-Alkoxy, -NR⁸R⁹ oder Heterocyclyl substituiert sind, wobei der Heterocylylring ein- oder mehrfach, gleich oder verschieden substituiert sein kann mit C₁-C₃-Alkyl oder -C(O)-R⁷,
wobei
R⁵ und R⁶ unabhängig voneinander stehen für Wasserstoff oder einen C₁-C₆ Alkylrest, der gegebenenfalls ein- oder mehrfach, gleich oder verschieden mit Hydroxy, -C₁-C₃-Alkoxy, -NR⁸R⁹ oder Heterocyclyl substituiert ist, wobei der Heterocylylring ein- oder mehrfach, gleich oder verschieden substituiert sein kann mit C₁-C₃-Alkyl oder - C(O)-R⁷, oder
R⁵ und R⁶ bilden alternativ gemeinsam mit dem Stickstoffatom einen 5- bis 7-gliedrigen Ring, der gegebenenfalls zusätzlich zum Stickstoffatom ein weiteres Heteroatom enthält und der gegebenenfalls mit C₁-C₆-Alkyl und/oder mit -C(O)-R⁷ ein- oder mehrfach, gleich oder verschieden substituiert ist, und
R⁷ für einen C₁-C₆ Alkylrest steht, und
R⁸, R⁹, R¹⁰ unabhängig voneinander stehen für Wasserstoff oder einen C₁-C₆ Alkylrest, und
R¹¹ und R¹² unabhängig voneinander stehen für Wasserstoff oder einen C₁-C₆ Alkylrest, der gegebenenfalls ein- oder mehrfach, gleich oder verschieden mit Hydroxy, -C₁-C₃-Alkoxy, -NR⁸R⁹ oder Heterocyclyl substituiert ist, wobei der Heterocylylring ein- oder mehrfach, gleich oder verschieden substituiert sein kann mit C₁-C₃-Alkyl oder - C(O)-R⁷,
sowie deren Salze, Enantiomere und Diastereomere.

2. Verbindungen der allgemeinen Formel (I) mit den Bausteinen A, B, C und D, in der
die Bausteine B und D in ortho-Stellung zueinander stehen und
Q₁ für einen Pyrazolyl-, Thienyl-, Imidazolyl-, oder 1,2,4-Oxadiazolylring steht,
R¹ und R² unabhängig voneinander stehen für
(i) Wasserstoff, Hydroxy, Halogen, Cyano, -CF₃, -NR⁵R⁶ oder
(ii) einen C₁-C₆-Alkyl-, C₁-C₆-Alkoxy-, C₁-C₆-Fluoralkyl- oder einen C₁-C₆-Fluoralkoxyrest oder
(iii) -SO₂-R⁷,
Q₂ für einen Thienyl-, Thiazolyl-, Furanyl-, Pyrrolyl-, Oxazolyl-, Imidazolyl-, Pyrazolyl-, Isoxazolyl, Isothiazolyl, 1,2,3-Triazolyl, Pyridinyl-, Pyridazinyl-, Pyrimidinyl-, Pyrazinyl-, 1,2,3,4-Tetrahydro-isochinolinyl-, 1,2,3,4-Tetrahydro-chinolinyl-, 2,3-Dihydro-1H-indolyl-, 2,3-Dihydro-1H-isoindolyl-, 4,5,6,7-Tetrahydro-thieno[2,3-c]pyridinyl-, 4,5,6,7-Tetrahydro-thieno[3,2-c]pyridinyl-, 2,3-Dihydro-benzofuranyl-, Benzo[1,3]dioxolyl-, 2,3-Dihydro-benzo[1,4]dioxinyl-, 1,2,3,4-Tetrahydro-chinoxalinyl - oder einen 3,4-Dihydro-2H-benzo[1,4]oxazinylring steht,
R³ und R⁴ unabhängig voneinander stehen für
(i) Wasserstoff, Halogen, -NR¹¹R¹² oder
(ii) einen C₁-C₆-Alkyl- oder C₁-C₆-Alkoxyrest, jeweils gegebenenfalls mit Morpholin oder-NR⁸R⁹ substituiert, wobei
R⁵ und R⁶ unabhängig voneinander stehen für Wasserstoff oder einen C₁-C₆ Alkylrest, der gegebenenfalls ein- oder mehrfach, gleich oder verschieden mit Hydroxy, -NR⁸R⁹, oder C₁-C₃-Alkoxy substituiert sein kann, und
R⁷ für einen C₁-C₄ Alkylrest steht, und
R⁸ und R⁹ unabhängig voneinander stehen für Wasserstoff oder einen C₁-C₄ Alkylrest,
R¹¹ und R¹² unabhängig voneinander stehen für Wasserstoff oder einen C₁-C₆ Alkylrest, der gegebenenfalls ein- oder mehrfach, gleich oder verschieden mit Morpholin oder durch -N(CH₃)₂, -NH-CH₃ oder -NH-C₂H₅ substituiert sein kann,
sowie deren Salze, Enantiomere und Diastereomere.

3. Verbindungen der allgemeinen Formel (I) mit den Bausteinen A, B, C und D gemäß Anspruch 1 oder 2, in der
Q₁ für einen Pyrazolyl- oder Thienylring steht,
sowie deren Salze, Enantiomere und Diastereomere.

4. Verbindungen der allgemeinen Formel (I) mit den Bausteinen A, B, C und D gemäß einem der Ansprüche 1 bis 3, in der
R¹ und R² unabhängig voneinander stehen für
(i) Wasserstoff, oder
(ii) einen C₁-C₆-Alkylrest,
sowie deren Salze, Enantiomere und Diastereomere.

5. Verbindungen der allgemeinen Formel (I) mit den Bausteinen A, B, C und D gemäß einem der Ansprüche 1 bis 4, in der
Q₂ für einen Pyrazolylring steht,
sowie deren Salze, Enantiomere und Diastereomere.

6. Verbindungen der allgemeinen Formel (I) mit den Bausteinen A, B, C und D gemäß einem der Ansprüche 1 bis 5, in der
R³ und R⁴ unabhängig voneinander stehen für Wasserstoff oder C₁-C₆-Alkyl,
sowie deren Salze, Enantiomere und Diastereomere.

7. Verbindungen der allgemeinen Formel (I) mit den Bausteinen A, B, C und D in der
die Bausteine B und D in ortho-Stellung zueinander stehen und
Q₁ für einen Pyrazolyl- oder Thienylring steht,
R¹ und R² unabhängig voneinander stehen für
(i) Wasserstoff
(ii) einen C₁-C₆-Alkylrest
Q₂ für einen Pyrazolylring steht,
R³ und R⁴ unabhängig voneinander stehen für Wasserstoff oder C₁-C₆-Alkyl,
sowie deren Salze, Enantiomere und Diastereomere.

8. Verbindung nach einem der Ansprüche 1 bis 7, nämlich
2-(1 H-Pyrazol-4-yl)-thiazol-4-carbonsäure (3-carbamoyl-1-methyl-1 H-pyrazol-4-yl)-amid,

9. Verfahren zur Herstellung von Verbindungen gemäß einem der Ansprüche 1 bis 8 **gekennzeichnet durch** die Schritte
a) Umsetzung von 2-Bromthiazol-4-carbonsäure mit Intermediaten der Formel (II) **durch** ein Amidkupplungsreagenz 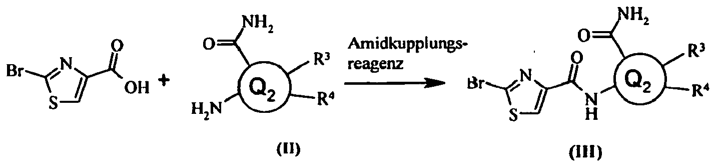
b) Umsetzung der Intermediate der Formel (III) mit Boronsäuren oder Boronsäurepinakolestern im Rahmen einer Suzuki-Miyaura-Reaktion zu Verbindungen der Formel (I) 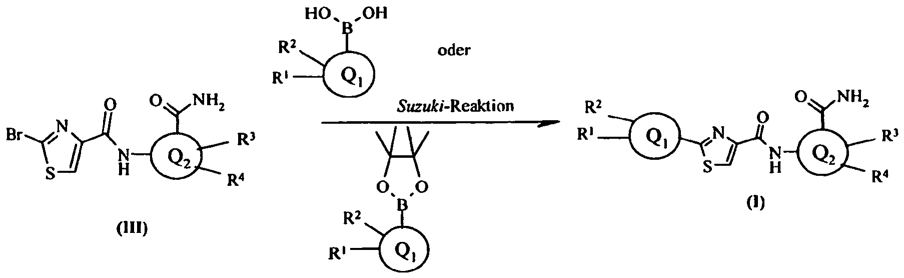
wobei
Q₁, Q₂, R¹, R², R³ und R⁴ die in der allgemeinen Formel (I) gemäß der Ansprüche 1 bis 7 angegebenen Bedeutungen besitzen.

10. Verbindungen gemäß einem der Ansprüche 1 bis 8 zur Verwendung als Arzneimittel.

11. Verwendung einer Verbindung gemäß einem der Ansprüche 1 bis 8 zur Herstellung eines Arzneimittel zur Behandlung von Erkrankungen, die mit entzündlichen, allergischen und / oder proliferativen Prozessen einhergehen.

12. Verbindungen gemäß einem der Ansprüche 1 bis 8 zur Verwendung als Arzneimittel gegen Erkrankungen, die mit entzündlichen, allergischen und / oder proliferativen Prozessen einhergehen.

13. Pharmazeutische Formulierung enthaltend eine Verbindung gemäß einem der Ansprüche 1 bis 8.

## Claims

1. Compounds of the general formula (I) with building blocks A, B, C and D 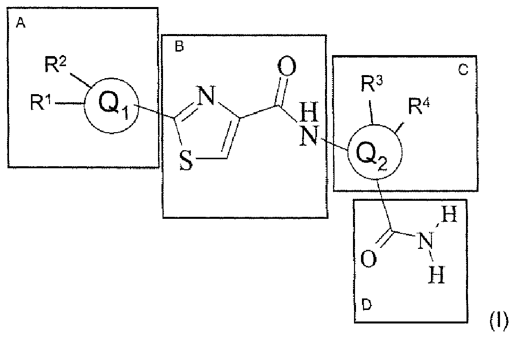 in which
the building blocks Band D are in ortho position relative to one another, and
Q₁ is a heteroaryl ring having 5 ring atoms,
R¹ and R² are independently of one another
(i) hydrogen, hydroxy, nitro, halogen, cyano, -CF₃, -NR⁵R⁶ or
(ii) -C(O)-R¹⁰, -C(O)-R⁷, -C(O)-C₁-C₃- fluoroalkyl, -C(O)-NR⁵R⁶, -NH-C(O)-R⁷ or
(iii) a C₁-C₆-alkyl, C₂-C₆-alkenyl, C₂-C₆- alkynyl, C₁-C₆-alkoxy, C₁-C₆- fluoroalkyl or a C₁-C₆-fluoroalkoxy radical,
in each case optionally substituted one or more times, identically or differently, by C₁-C₃-alkoxy, hydroxy, -C(O)-R¹⁰ or -NR⁸R⁹, or
(iv) -O-SO₂-NR⁵R⁶, -SO₂-R⁷, -SO₂-NR⁵R⁶,
Q₂ is a heteroaryl or a hydrogenated bicyclic heteroaryl ring,
R³ and R⁴ are independently of one another
(i) hydrogen, halogen or -NR¹¹R¹², or
(ii) a C₁-C₆-alkyl or C₁-C₆-alkoxy radical which are in each case optionally substituted one or more times, identically or differently, by hydroxy, C₁-C₃-alkoxy, -NR⁸R⁹ or heterocyclyl, where the heterocyclyl ring may be substituted one or more times, identically or differently, by C₁-C₃-alkyl or -C(O)-R⁷,
where
R⁵ and R⁶ are independently of one another hydrogen or a C₁-C₆-alkyl radical which is optionally substituted one or more times, identically or differently, by hydroxy, - C₁-C₃-alkoxy, -NR⁸R⁹ or heterocyclyl, where the heterocyclyl ring may be substituted one or more times, identically or differently, by C₁-C₃-alkyl or -C(O)-R⁷, or
R⁵ and R⁶ form alternatively together with the nitrogen atom a 5- to 7-membered ring which optionally comprises a further heteroatom in addition to the nitrogen atom, and which is optionally substituted one or more times, identically or differently, by C₁-C₆-alkyl and/or by - C(O)-R⁷, and
R⁷ is a C₁-C₆-alkyl radical, and
R⁸, R⁹, R¹⁰ are independently of one another hydrogen or a C₁-C₆-alkyl radical, and
R¹¹ and R¹² are independently of one another hydrogen or a C₁-C₆-alkyl radical which is optionally substituted one or more times, identically or differently, by hydroxy, - C₁-C₃-alkoxy, -NR⁸R⁹ or heterocyclyl, where the heterocyclyl ring may be substituted one or more times, identically or differently, by C₁-C₃-alkyl or -C(O)-R⁷,
and the salts, enantiomers and diastereomers thereof.

2. Compounds of the general formula (I) with building blocks A, B, C and D,
in which
the building blocks B and D are in ortho position relative to one another, and
Q₁ is a pyrazolyl, thienyl, imidazolyl or 1,2,4-oxadiazolyl ring,
R¹ and R² are independently of one another
(i) hydrogen, hydroxy, halogen, cyano, -CF₃, -NR⁵R⁶ or
(ii) a C₁-C₆-alkyl, C₁-C₆-alkoxy, C₁-C₆- fluoroalkyl or a C₁-C₆-fluoroalkoxy radical or
(iii) -SO₂R⁷,
Q₂ is a thienyl, thiazolyl, furanyl, pyrrolyl, oxazolyl, imidazolyl, pyrazolyl, isoxazolyl, isothiazolyl, 1,2,3-triazolyl, pyridinyl, pyridazinyl, pyrimidinyl, pyrazinyl, 1,2,3,4-tetrahydroisoquinolinyl, 1,2,3,4-tetra- hydroquinolinyl, 2,3-dihydro-1H-indolyl, 2,3-dihydro-1H-isoindolyl, 4,5,6,7-tetrahydrothieno[2,3-c]pyridinyl, 4,5,6,7-tetrahydrothieno[3,2-c]pyridinyl, 2,3-dihydrobenzofuranyl, benzo[1,3]dioxolyl, 2,3-dihydro- benzo[1,4]dioxinyl, 1,2,3,4-tetrahydroquinoxalinyl or a 3,4-dihydro-2H-benzo[1,4]oxazinyl ring,
R³ and R⁴ are independently of one another
(i) hydrogen, halogen, -NR¹¹R¹² or
(ii) a C₁-C₆-alkyl or C₁-C₆-alkoxy radical,
in each case optionally substituted by morpholine or -NR⁸R⁹,
where
R⁵ and R⁶ are independently of one another hydrogen or a C₁-C₆ alkyl radical which may optionally be substituted one or more times, identically or differently, by hydroxy, -NR⁸R⁹ or C₁-C₃-alkoxy, and
R⁷ is a C₁-C₄ alkyl radical, and
R⁸ and R⁹ are independently of one another hydrogen or a C₁-C₄ alkyl radical,
R¹¹ and R¹² are independently of one another hydrogen or a C₁-C₆ alkyl radical which may optionally be substituted one or more times, identically or differently, by morpholine or by - N(CH₃)₂, -NH-CH₃ or -NH-C₂H₅,
and the salts, enantiomers and diastereomers thereof.

3. Compounds of the general formula (I) with building blocks A, B, C and D according to Claim 1 or 2, in which
Q₁ is a pyrazolyl or thienyl ring,
and the salts, enantiomers and diastereomers thereof.

4. Compounds of the general formula (I) with building blocks A, B, C and D according to any of Claims 1 to 3, in which
R¹ and R² are independently of one another
(i) hydrogen, or
(ii) a C₁-C₆-alkyl radical,
and the salts, enantiomers and diastereomers thereof.

5. Compounds of the general formula (I) with building blocks A, B, C and D according to any of Claims 1 to 4, in which
Q₂ is a pyrazolyl ring,
and the salts, enantiomers and diastereomers thereof.

6. Compounds of the general formula (I) with building blocks A, B, C and D according to any of Claims 1 to 5, in which
R³ and R⁴ are independently of one another hydrogen or C₁-C₆-alkyl,
and the salts, enantiomers and diastereomers thereof.

7. Compounds of the general formula (I) with building blocks A, B, C and D in which
the building blocks B and D are in ortho position relative to one another, and
Q₁ is a pyrazolyl or thienyl ring,
R¹ and R² are independently of one another
(i) hydrogen
(ii) a C₁-C₆-alkyl radical
Q₂ is a pyrazolyl ring,
R³ and R⁴ are independently of one another hydrogen or C₁-C₆-alkyl,
and the salts, enantiomers and diastereomers thereof.

8. Compound according to any of Claims 1 to 7, namely 2-(1H-pyrazol-4-yl)thiazole-4-(3-carbamoyl-1-methyl)-1H-pyrazol-4-yl)carboxamide).

9. Process for preparing compounds according to any of Claims 1 to 8 **characterized by** the steps
a) reaction of 2-bromothiazole-4-carboxylic acid with intermediates of the formula (II) by an amide coupling reagent 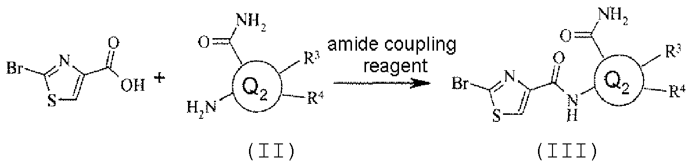
b) reaction of the intermediates of the formula (III) with boronic acids or boronic acid pinacol esters in a *Suzuki-Miyaura* reaction to give compounds of the formula (I) 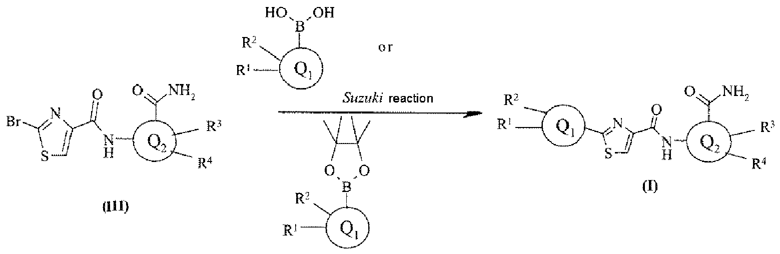 where
Q₁, Q₂, R¹, R², R³ and R⁴ have the meanings indicated in the general formula (I) according to Claims 1 to 7.

10. Compounds according to any of Claims 1 to 8 for use as pharmaceuticals.

11. Use of a compound according to any of Claims 1 to 8 for producing a pharmaceutical for the treatment of disorders associated with inflammatory, allergic and/or proliferative processes.

12. Compounds according to any of Claims 1 to 8 for use as pharmaceuticals for treating disorders associated with inflammatory, allergic and/or proliferative processes.

13. Pharmaceutical formulation comprising a compound according to any of Claims 1 to 8.

## Revendications

1. Composés de formule générale (I) présentant les éléments A, B, C et D, 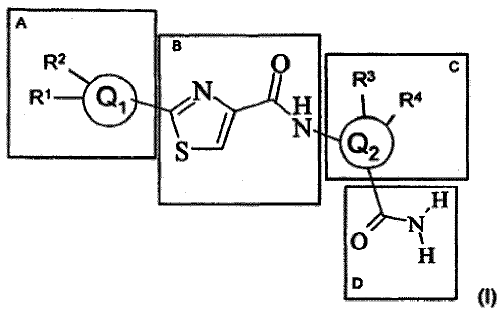 dans laquelle les éléments B et D se trouvent en position ortho l'un par rapport à l'autre et
Q₁ représente un cycle hétéroaryle comprenant 5 atomes de cycle,
R¹ et R² représentent, indépendamment l'un de l'autre,
(i) hydrogène, hydroxy, nitro, halogène, cyano, -CF₃, -NR⁵R⁶ ou
(ii) -C(O)-OR¹⁰, -C(O)-R⁷, -C(O)-C₁-C₃- fluoroalkyle, -C(O)-NR⁵R⁶, -NH-C(O)-R⁷ ou
(iii) un radical C₁-C₆-alkyle, C₂-C₆-alcényle, C₂-C₆-alcynyle, C₁-C₆-alcoxy, C₁-C₆- fluoroalkyle ou C₁-C₆-fluoroalcoxy,
à chaque fois le cas échéant monosubstitué ou polysubstitué, de manière identique ou différente, par -C₁-C₃-alcoxy, hydroxy, -C(O)-OR¹⁰ ou -NR⁸R⁹, ou
(iv) -O-SO₂-NR⁵R⁶, -SO₂-R⁷, -SO₂-NR⁵R⁶,
Q₂ représente un cycle hétéroaryle ou un cycle hétéroaryle bicyclique hydrogéné,
R³ et R⁴ représentent, indépendamment l'un de l'autre,
(i) hydrogène, halogène ou -NR¹¹R¹², ou
(ii) un radical C₁-C₆-alkyle ou C₁-C₆-alcoxy, à chaque fois le cas échéant monosubstitué ou polysubstitué, de manière identique ou différente, par hydroxy, C₁-C₃-alcoxy, -NR⁸R⁹ ou hétérocyclyle, le cycle hétérocyclyle pouvant être monosubstitué ou polysubstitué, de manière identique ou différente, par C₁-C₃- alkyle ou -C(O)-R⁷,
où
R⁵ et R⁶ représentent, indépendamment l'un de l'autre, hydrogène ou un radical C₁-C₆-alkyle, qui est le cas échéant monosubstitué ou polysubstitué, de manière identique ou différente, par hydroxy, -C₁-C₃-alcoxy, -NR⁸R⁹ ou hétérocyclyle, le cycle hétérocyclyle pouvant être monosubstitué ou polysubstitué, de manière identique ou différente, par C₁-C₃-alkyle ou -C(O)-R⁷, ou
R⁵ et R⁶ forment, en variante, ensemble avec l'atome d'azote, un cycle de 5 à 7 chaînons, qui contient le cas échéant, en plus de l'atome d'azote, un autre hétéroatome et qui est le cas échéant monosubstitué ou polysubstitué, de manière identique ou différente, par C₁-C₆-alkyle et/ou par -C(O)-R⁷, et
R⁷ représente un radical C₁-C₆-alkyle, et
R⁸, R⁹, R¹⁰ représentent, indépendamment l'un de l'autre, hydrogène ou un radical C₁-C₆-alkyle, et
R¹¹ et R¹² représentent, indépendamment l'un de l'autre, hydrogène ou un radical C₁-C₆-alkyle, qui est le cas échéant monosubstitué ou polysubstitué, de manière identique ou différente, par hydroxy, -C₁-C₃-alcoxy, -NR⁸R⁹ ou hétérocyclyle, le cycle hétérocyclyle pouvant être monosubstitué ou polysubstitué, de manière identique ou différente, par C₁-C₃-alkyle ou -C(O)-R⁷,
ainsi que leurs sels, énantiomères et diastéréo-isomères.

2. Composés de formule générale (I) présentant les éléments A, B, C et D, dans laquelle
les éléments B et D se trouvent en position ortho l'un par rapport à l'autre et
Q₁ représente un cycle pyrazolyle, thiényle, imidazolyle ou 1,2,4-oxadiazolyle,
R¹ et R² représentent, indépendamment l'un de l'autre,
(i) hydrogène, hydroxy, halogène, cyano, -CF₃, -NR⁵R⁶ ou
(ii) un radical C₁-C₆-alkyle, C₁-C₆-alcoxy, C₁-C₆-fluoroalkyle ou C₁-C₆-fluoroalcoxy ou
(iii) -SO₂-R⁷,
Q₂ représente un cycle thiényle, thiazolyle, furannyle, pyrrolyle, oxazolyle, imidazolyle, pyrazolyle, isoxazolyle, isothiazolyle, 1,2,3-triazolyle, pyridinyle, pyridazinyle, pyrimidinyle, pyrazinyle, 1,2,3,4-tétrahydro-isoquinoléinyle, 1,2,3,4-tétrahydroquinoléinyle, 2,3-dihydro-1H-indolyle, 2,3-dihydro-1H-iso-indolyle, 4,5,6,7-tétrahydrothiéno[2,3-c]pyridinyle, 4,5,6,7-tétrahydrothiéno[3,2-c]pyridinyle, 2,3-dihydrobenzofurannyle, benzo[1,3]dioxolyle, 2,3-dihydrobenzo[1,4]dioxinyle, 1,2,3,4-tétrahydroquinoxalinyle ou 3,4-dihydro-2H-benzo[1,4]oxazinyle,
R³ et R⁴ représentent, indépendamment l'un de l'autre,
(i) hydrogène, halogène, -NR¹¹R¹² ou
(ii) un radical C₁-C₆-alkyle ou C₁-C₆-alcoxy,
à chaque fois le cas échéant substitué par morpholine ou -NR⁸R⁹,
où
R⁵ et R⁶ représentent, indépendamment l'un de l'autre, hydrogène ou un radical C₁-C₆-alkyle, qui peut le cas échéant être monosubstitué ou polysubstitué, de manière identique ou différente, par hydroxy, -NR⁸R⁹ ou -C₁-C₃-alcoxy, et
R⁷ représente un radical C₁-C₄-alkyle, et
R⁸ et R⁹ représentent, indépendamment l'un de l'autre, hydrogène ou un radical C₁-C₄-alkyle, et
R¹¹ et R¹² représentent, indépendamment l'un de l'autre, hydrogène ou un radical C₁-C₆-alkyle, qui peut le cas échéant être monosubstitué ou polysubstitué, de manière identique ou différente, par morpholine ou par -N(CH₃)₂, -NH-CH₃ ou -NH-C₂H₅,
ainsi que leurs sels, énantiomères et diastéréo-isomères.

3. Composés de formule générale (I) présentant les éléments A, B, C et D selon la revendication 1 ou 2, dans laquelle
Q₁ représente un cycle pyrazolyle ou thiényle,
ainsi que leurs sels, énantiomères et diastéréo-isomères.

4. Composés de formule générale (I) présentant les éléments A, B, C et D selon l'une quelconque des revendications 1 à 3, dans laquelle
R¹ et R² représentent, indépendamment l'un de l'autre,
(i) hydrogène, ou
(ii) un radical C₁-C₆-alkyle,
ainsi que leurs sels, énantiomères et diastéréo-isomères.

5. Composés de formule générale (I) présentant les éléments A, B, C et D selon l'une quelconque des revendications 1 à 4, dans laquelle
Q₂ représente un cycle pyrazolyle,
ainsi que leurs sels, énantiomères et diastéréo-isomères.

6. Composés de formule générale (I) présentant les éléments A, B, C et D selon l'une quelconque des revendications 1 à 5, dans laquelle
R³ et R⁴ représentent, indépendamment l'un de l'autre, hydrogène ou C₁-C₆-alkyle,
ainsi que leurs sels, énantiomères et diastéréo-isomères.

7. Composés de formule générale (I) présentant les éléments A, B, C et D,
dans laquelle les éléments B et D se trouvent en position ortho l'un par rapport à l'autre et
Q₁ représente un cycle pyrazolyle ou thiényle,
R¹ et R² représentent, indépendamment l'un de l'autre,
(i) hydrogène
(ii) un radical C₁-C₆-alkyle,
Q₂ représente un cycle pyrazolyle,
R³ et R⁴ représentent, indépendamment l'un de l'autre, hydrogène ou C₁-C₆-alkyle,
ainsi que leurs sels, énantiomères et diastéréo-isomères.

8. Composé selon l'une quelconque des revendications 1 à 7, à savoir le (3-carbamoyl-1-méthyl-1H-pyrazol-4-yl)-amide de l'acide 2-(1H-pyrazol-4-yl)-thiazole-4-carboxylique.

9. Procédé pour la préparation de composés selon au moins l'une quelconque des revendications 1 à 8, **caractérisé par** les étapes
a) transformation d'acide 2-bromothiazole-4-carboxylique avec des intermédiaires de formule (II) par un réactif de couplage d'amide 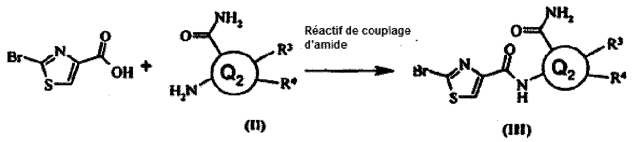
b) transformation de l'intermédiaire de formule (III) avec des acides boroniques ou des esters de pinacol d'acide boronique dans le cadre d'une réaction de Suzuki-Miyaura en composés de formule (I) 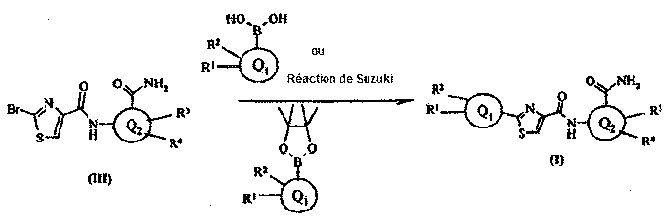 où
Q₁, Q₂, R¹, R², R³ et R⁴ ont les significations indiquées dans la formule générale (I) selon les revendications 1 à 7.

10. Composés selon l'une quelconque des revendications 1 à 8 destinés à une utilisation comme médicament.

11. Utilisation d'un composé selon l'une quelconque des revendications 1 à 8 pour la préparation d'un médicament destiné au traitement de maladies, qui vont de pair avec des processus inflammatoires, allergiques et/ou de prolifération.

12. Composés selon l'une quelconque des revendications 1 à 8 destinés à une utilisation comme médicament contre des maladies, qui vont de pair avec des processus inflammatoires, allergiques et/ou de prolifération.

13. Formulation pharmaceutique contenant un composé selon l'une quelconque des revendications 1 à 8.
